(19) Europäisches Patentamt
European Patent Office
Office européen des brevets

(11) Veröffentlichungsnummer: **0 391 132 A1**

(12) # EUROPÄISCHE PATENTANMELDUNG

(21) Anmeldenummer: **90105293.6**

(22) Anmeldetag: **21.03.90**

(51) Int. Cl.5: **C07D 487/08, C07D 471/04, C07D 498/04, C07D 487/04, C07D 401/04, A61K 31/47, //(C07D487/08,209:00,209:00), (C07D487/08,243:00,209:00), (C07D471/04,221:00,209:00), (C07D498/04,263:00,209:00), (C07D498/04,265:00,209:00), (C07D487/04,209:00,209:00)**

(30) Priorität: **03.04.89 DE 3910663**

(43) Veröffentlichungstag der Anmeldung:
**10.10.90 Patentblatt 90/41**

(84) Benannte Vertragsstaaten:
**AT BE CH DE DK ES FR GB GR IT LI LU NL SE**

(71) Anmelder: **BAYER AG**

**D-5090 Leverkusen 1 Bayerwerk(DE)**

(72) Erfinder: **Schriewer, Michael, Dr.**
**Am Thelen Siefen 1A**
**D-5068 Odenthal(DE)**
Erfinder: **Grohe, Klaus, Dr.**
**Am Wasserturm 10**
**D-5068 Odenthal(DE)**
Erfinder: **Krebs, Andreas, Dr.**
**Am Gartenfeld 70**
**D-5068 Odenthal(DE)**
Erfinder: **Petersen, Uwe, Dr.**
**Auf dem Forst 4**
**D-5090 Leverkusen 1(DE)**
Erfinder: **Schenke, Thomas, Dr.**
**Mühlenstrasse 113**
**D-5060 Bergisch-Gladbach 2(DE)**
Erfinder: **Haller, Ingo, Dr.**
**Dornröschenweg 4**
**D-5600 Wuppertal 1(DE)**
Erfinder: **Metzger, Karl Georg, Dr.**
**Pahlkestrasse 75**
**D-5600 Wuppertal 1(DE)**
Erfinder: **Endermann, Rainer, Dr.**
**In der Birken 152a**
**D-5600 Wuppertal 1(DE)**
Erfinder: **Zeiler, Hans-Joachim, Dr.**
**Elsbeekerstrasse 46**
**D-5620 Velbert 15(DE)**

(54) **5-Alkylchinoloncarbonsäuren.**

(57) Die Erfindung betrifft neue Chinolon- und Naphthyridincarbonsäurederivate der Formel (I)

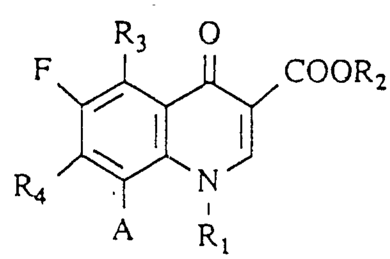

(I)

in welcher $R_1$, $R_2$, $R_3$, $R_4$ und A die in der Beschreibung angegebene Bedeutung haben und die in 5-

EP 0 391 132 A1

Stellung einen Alkyl bzw. einen substituierten Alkylrest tragen, Verfahren zu ihrer Herstellung sowie diese enthaltende antibakterielle Mittel und Futterzusatzstoffe.

## 5-Alkylchinoloncarbonsäuren

Die Erfindung betrifft neue Chinoloncarbonsäurederivate, die in 5-Stellung einen Alkyl bzw. einen substituierten Alkylrest tragen, Verfahren zu ihrer Herstellung sowie diese enthaltende antibakterielle Mittel und Futterzusatzstoffe.

Es wurde gefunden, daß Chinolonsäure-Derivate der Formel (I)

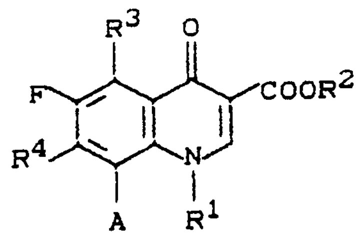

(I)

in welcher

$R^1$ für gegebenenfalls durch Hydroxy, Halogen, $C_1$-$C_3$-Alkoxy oder $C_1$-$C_3$-Alkylthio substiutiertes gerad kettiges oder verzweigtes $C_1$-$C_4$-Alkyl, für gegebenenfalls durch Halogen oder $C_1$-$C_3$-Alkyl substituiertes $C_3$-$C_6$-Cycloalkyl, $C_1$-$C_4$-Alkenyl, ferner für $C_1$-$C_3$-Alkoxy, Amino, Monoalkylamino mit 1-3 C-Atomen, Dialkylamino mit 2-6 C-Atomen oder für gegebenenfalls durch Halogen substituiertes Phenyl steht,

$R^2$ für Wasserstoff, Alkyl mit 1 bis 4 Kohlenstoffatomen oder (5-Methyl-2-oxo-1,3-dioxol-4-yl)-methyl steht

$R^3$ $C_1$-$C_4$-Alkyl bedeutet,

$R^4$ für einen im Ringsystem gegebenenfalls durch Hydroxy oder Methyl substituierten Rest der Formel

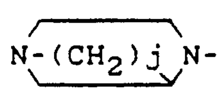 , 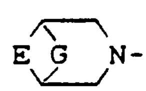 , 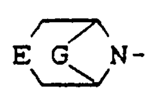 , 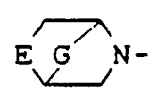 ,

in welcher

E für $R^5$-N, O, S,

G für $-(CH_2)_j-$, $-CH_2-O-CH_2-$, $-CH_2-S-CH_2-$, $-CH_2-S-$,

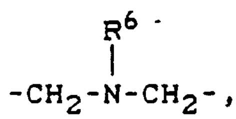

j für 1, 2 oder 3,

$R^5$ für Wasserstoff, gegebenenfalls durch Hydroxy substituiertes Alkyl, Alkenyl oder Alkinyl mit 1 bis 4 Kohlenstoffatomen, gegebenenfalls durch Nitro oder Amino substituiertes Benzyl, Oxoalkyl mit 2 bis 4 Kohlenstoffatomen oder (5-Methyl-2-oxo-1,3-dioxol-4-yl)-methyl,

$R^6$ für Wasserstoff oder Methyl steht,

$R^4$ ferner für einen Rest der Formel

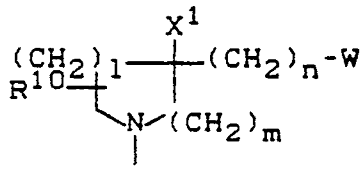

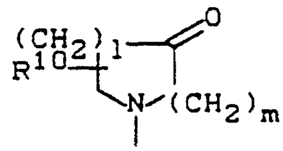

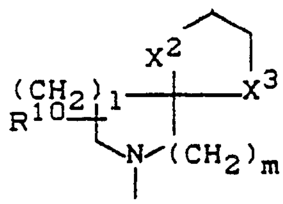

,

steht worin,
l für 0, 1 oder 2,
m für 1 oder 2 steht, wobei l + m gemeinsam 1, 2 oder 3 sein können,
n für 1 oder 2,
W für

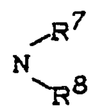

,

$OR^9$, $SR^9$, Halogen oder Wasserstoff,
$X^1$ für

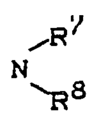

,

$OR^9$, $SR^9$, Halogen, CN, $CONH_2$, COOH, oder $C_1$-$C_4$-alkyl,
$X^2$ und $X^3$ gleich oder verschieden sein können und für Sauerstoff oder N-$CH_3$ stehen,
$R^7$ für Wasserstoff, $C_1$-$C_3$-Alkyl, Allyl oder Propargyl und
$R^8$ für Wasserstoff, $C_1$-$C_3$-Alkyl oder $C_3$-$C_6$-Cycloalkyl steht,
wobei
$R^7$ + $R^8$ auch gemeinsam die Gruppen -$CH_2CH_2$-O-$CH_2CH_2$- oder -$(CH_2)_k$-, in welcher k für 3, 4, oder 5 stehen kann, bedeutet und
$R^9$ für Wasserstoff, $C_1$-$C_3$-Alkyl oder $C_1$-$C_3$-Acyl,
$R^{10}$ für Wasserstoff oder $C_1$-$C_3$-Alkyl steht,
$R^4$ ebenfalls einen Rest der Struktur

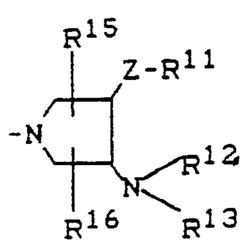

,

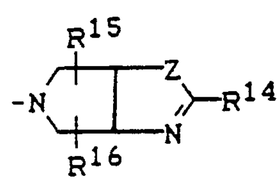

,

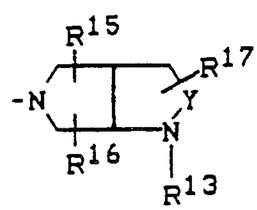

bedeutet, worin
$R^{11}$ für H, $C_1$-$C_3$-Alkyl, $C_1$-$C_2$-Acyl
$R^{12}$ für H, $C_1$-$C_3$-Alkyl, OH, $OCH_3$, wobei $R^{11}$ und $R^{12}$ gemeinsam auch eine gegebenenfalls durch Methyl ein- oder zweifach substituierte $C_1$-$C_2$-Alkylenbrücke bedeuten kann,
$R^{13}$ für H, $C_1$-$C_3$-Alkyl, Aryl, Heteroaryl, Benzyl, $C_1$-$C_4$-Alkoxycarbonyl , $C_1$-$C_4$-Acyl oder (5-Methyl-2-oxo-1,3-dioxol-4-yl)-methyl,

$R^{14}$ für H oder $C_1$-$C_4$-Alkyl,
$R^{15}$ für H oder $CH_3$ oder Phenyl,
$R^{16}$ für H oder $CH_3$ oder Phenyl,
$R^{17}$ für H oder $CH_3$,
Y für O, $CH_2$, $CH_2CH_2$ oder $CH_2$-O stehen kann, wobei die Verknüpfung der $CH_2$-O-Gruppe zum Stickstoff sowohl über O als auch über $CH_2$ erfolgen kann,
Z für O oder S stehen kann,
A für Wasserstoff, Halogen, Methyl, Cyano oder Nitro steht oder auch gemeinsam mit $R^1$ eine Brücke der Struktur
$-O-CH_2\ \overset{*}{C}H-CH_3$ , $-S-CH_2-\ \overset{*}{C}H-CH_3$ oder $-CH_2CH_2-\ \overset{*}{C}H-CH_3$
mit R- bzw. S-Konfiguration bilden kann
und deren pharmazeutisch verwendbaren Hydrate und Säureadditionssalze sowie die Alkali-, Erdalkali-, Silber-und Guanidiniumsalze der zugrundeliegenden Carbonsäuren eine hohe antibakterielle Wirkung insbesondere im gram-positiven Bereich aufweisen.

Sie eignen sich daher als Wirkstoffe für die Human- und Veterinärmedizin, wobei zur Veterinärmedizin auch die Behandlung von Fischen zur Therapie oder Vorbeugung bakterieller Infektionen zu zählen ist.

Bevorzugt sind diejenigen Verbindungen der Formel (I), in denen
$R^1$ für Ethyl, Isopropyl, Cyclopropyl, Vinyl, t-Butyl, 2-Hydroxyethyl, 2-Fluorethyl, Amino, Methylamino, Phenyl, 4-Fluorphenyl oder 2,4-Difluorphenyl,
$R^2$ für Wasserstoff, Alkyl mit 1 bis 3 Kohlenstoffatomen oder (5-Methyl-2-oxo-1,3-dioxol-4-yl)-methyl,
$R^3$ für $C_1$-$C_3$-Alkyl steht
$R^4$ für einen im Ringsystem gegebenenfalls durch Methyl substituierten Rest der Formel

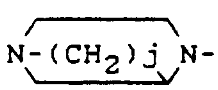 , 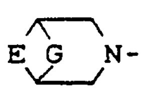 , 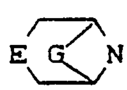 , 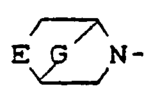 ,

in welcher
E für $R^5$-N, O,
G für $-(CH_2)_j-$, $-CH_2-O-CH_2-$,

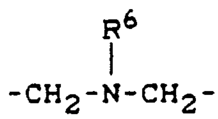

j für 1, 2 oder 3,
$R^5$ für Wasserstoff, gegebenenfalls durch Hydroxy substituiertes Alkyl, Alkenyl oder Alkinyl mit 1 bis 3 Kohlenstoffatomen, gegebenenfalls durch Nitro oder Amino substituiertes Benzyl oder Oxoalkyl mit 2 bis 4 Kohlenstoffatomen und
$R^6$ für Wasserstoff oder Methyl steht,
$R^4$ ferner für einen Rest der Formel

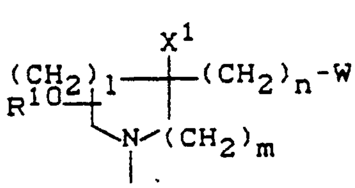

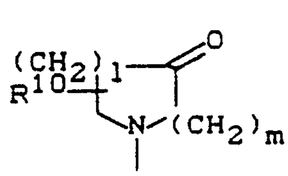

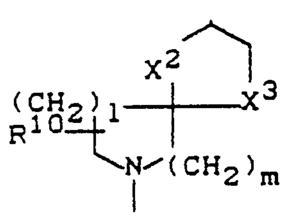 ,

steht, worin
l für 0, 1 oder 2,
m für 1 oder 2 steht, wobei l + m gemeinsam 1, 2 oder 3 sein können,
n für 1 oder 2,
W für

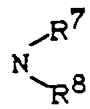 ,

$OR^9$ oder Wasserstoff,
X' für

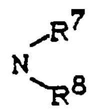 ,

$OR^9$ Fluor, Chlor oder $C_1$-$C_2$-Alkyl steht,
$X^2$ und $X^3$ gleich oder verschieden sein können und für Sauerstoff, Schwefel oder N-$CH_3$ stehen,
$R^7$ für Wasserstoff, $C_1$-$C_2$-Alkyl oder Acetyl,
$R^8$ für Wasserstoff oder $C_1$-$C_2$-Alkyl steht,
wobei
$R^7$ + $R^8$ auch gemeinsam die Gruppen -$CH_2CH_2$-O-$CH_2CH_2$- oder -$(CH_2)_k$-, in welcher k für 3, 4, oder 5 stehen kann, bedeutet,
$R^9$ für Wasserstoff, $C_1$-$C_2$-Alkyl oder Acetyl,
$R^{10}$ für Wasserstoff oder $C_1$-$C_2$-Alkyl steht,
$R^4$ ebenfalls einen Rest der Struktur

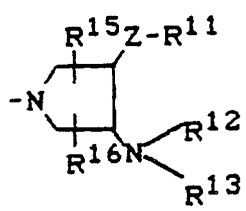 , 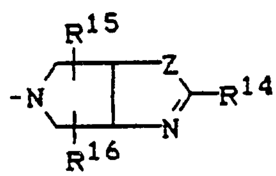 , 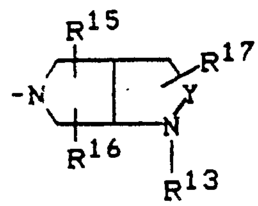

bedeutet, worin
$R^{11}$ für H, $C_1$-$C_3$-Alkyl, $C_1$-$C_2$-Acyl
$R^{12}$ für H, $C_1$-$C_3$-Alkyl, OH, $OCH_3$, wobei $R^{11}$ und $R^{12}$ gemeinsam auch eine gegebenenfalls durch Methyl ein- oder zweifach substituierte $C_1$-$C_2$-Alkylenbrücke bedeuten kann,
$R^{13}$ für H, $C_1$-$C_3$-Alkyl, Phenyl, Benzyl, $C_1$-$C_4$-Alkoxycarbonyl, $C_1$-$C_2$-Acyl oder (5-Methyl-2-oxo-1,3-dioxol-4-yl)-methyl,
$R^{14}$ für H oder $C_1$-$C_2$-Alkyl,
$R^{15}$ für H oder $CH_3$,
$R^{16}$ für H oder $CH_3$,
$R^{17}$ für H oder $CH_3$,
Y für O, $CH_2$, $CH_2CH_2$ oder $CH_2$-O stehen kann, wobei die Verknüpfung der $CH_2$-O-Gruppe zum Stickstoff sowohl über O als auch über $CH_2$ erfolgen kann,
Z für O stehen kann,
A für H, Fluor, Chlor, Methyl, Cyano oder Nitro steht oder auch gemeinsam mit R' eine Brücke der Struktur
-O-$CH_2$- $\overset{*}{C}H$-$CH_3$
mit R- bzw. S-Konfiguration bilden kann.

Besonders bevorzugt sind diejenigen Verbindungen der Formel (I), in denen
R' für Ethyl, Vinyl, t-Butyl, Cyclopropyl, 2-Hydroxyethyl, 2-Fluorethyl, Methylamino, 4-Fluorphenyl, 2,4-Difluorphenyl,
$R^2$ für Wasserstoff, Alkyl mit 1 bis 2 Kohlenstoffatomen,
$R^3$ für $C_1$-$C_3$-Alkyl steht

$R^4$ für einen im Ringsystem gegebenenfalls durch Methyl substituierten Rest der Formel

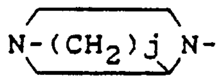 , 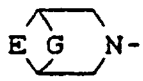 , 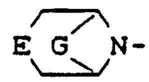 , 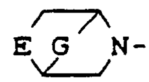 ,

in welcher
E für $R^5$-N,
G für $-(CH_2)_j-$,
j für 1 oder 2,
$R^5$ für Wasserstoff, gegebenenfalls durch Hydroxy substituiertes Alkyl, Alkenyl oder Alkinyl mit 1 bis 3 Kohlenstoffatomen, gegebenenfalls durch Nitro oder Amino substituiertes Benzyl oder Oxoalkyl mit 2 bis 4 Kohlenstoffatomen und
$R^6$ für Wasserstoff oder Methyl steht,
$R^4$ ferner für einen Rest der Formel

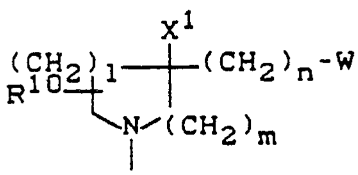 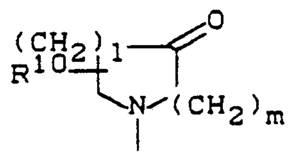

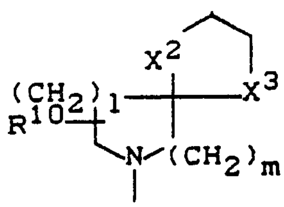 ,

steht, worin
l für 0, 1 oder 2,
m für 1 oder 2 steht, wobei l + m gemeinsam 1, 2 oder 3 sein können,
n für 1,
W für

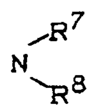 ,

$OR^9$ oder Wasserstoff,
$X^1$ für

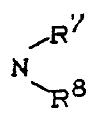 ,

$OR^9$, Chlor oder Methyl steht,
$X^2$ und $X^3$ gleich oder verschieden sein können und für Sauerstoff oder $N\text{-}CH_3$ stehen,
$R^7$ für Wasserstoff oder Methyl,
$R^8$ für Wasserstoff oder Methyl und
$R^9$ für Wasserstoff oder Methyl,
$R^{10}$ für Wasserstoff oder Methyl steht
$R^4$ außerdem einen Rest der Struktur

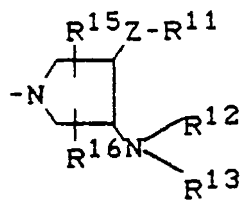, 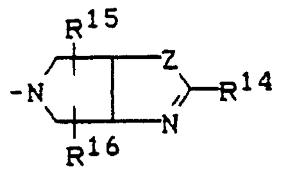, 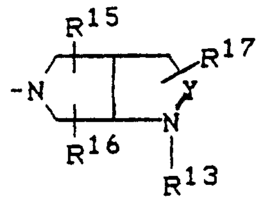

bedeutet, worin
$R^{11}$ für H, $C_1$-$C_2$-Alkyl, Acetyl,
$R^{12}$ für H, $C_1$-$C_2$-Alkyl, wobei $R^{11}$ und $R^{12}$ gemeinsam auch eine gegebenenfalls durch Methyl substituierte $C_1$-$C_2$-Alkylenbrücke bedeuten kann,
$R^{13}$ für H, $C_1$-$C_2$-Alkyl, Hydroxyethyl, Benzyl, $C_1$-$C_4$-Alkoxycarbonyl, $C_1$-$C_2$-Acyl,
$R^{14}$ für H oder $CH_3$,
$R^{15}$ für H oder $CH_3$,
$R^{16}$ für H oder $CH_3$,
$R^{17}$ für H oder $CH_3$,
Y für O, $CH_2$, $CH_2CH_2$ oder $CH_2$-O stehen kann, wobei die Verknüpfung der $CH_2$-O-Gruppen zum Stickstoff sowohl über O als auch über $CH_2$ erfolgen kann,
Z für O stehen kann,
A für H, Fluor oder Chlor steht, oder auch gemeinsam mit $R^1$ eine Brücke der Struktur -O-$CH_2$-$\overset{*}{C}H$-$CH_3$ mit R- bzw. S-Konfiguration bilden kann.

Weiterhin wurde gefunden, daß man die Verbindungen der Formel (I) erhält, wenn man Verbindungen der Formel (II)

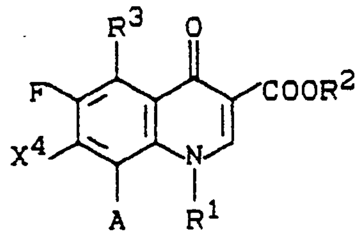

(II)

in welcher
$R^1$, $R^2$, $R^3$ und A die oben angegebene Bedeutung haben und
$X^4$ für Halogen, insbesondere Fluor oder Chlor steht mit Verbindungen der Formel (III)
$R^4$-H (III)
in welcher
$R^4$ die oben angegebene Bedeutung hat,
gegebenenfalls in Gegenwart von Säurefängern umsetzt und gegebenenfalls in $R^4$ enthaltene Schutzgruppen abspaltet.

Verwendet man beispielsweise 1-Cyclopropyl-6,7,8-trifluor-1,4-dihydro-5-methyl 4-oxo-3-chinolincarbonsäure und 1-Methyl-octahydropyrrolo[3,4-b]pyridin als Ausgangsstoffe, so kann der Reaktionsverlauf durch folgendes Formelschema wiedergegeben werden:

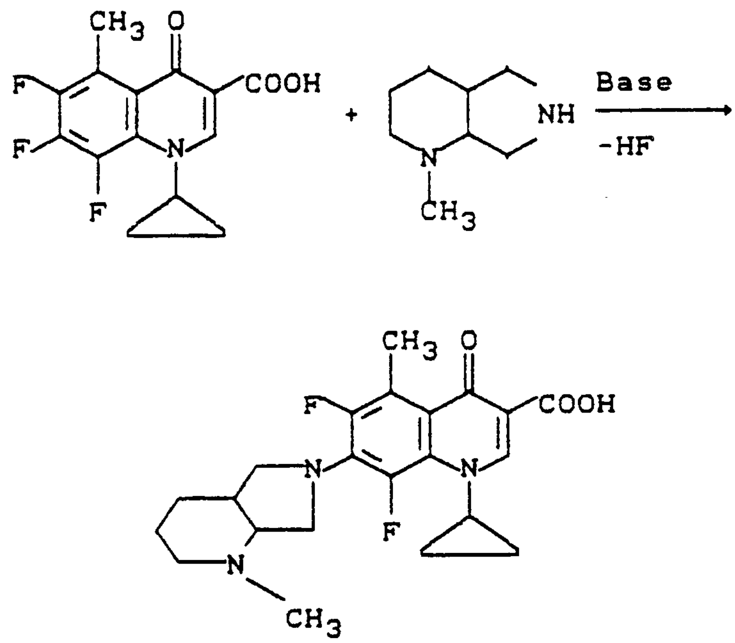


Verwendet man beispielsweise 8-Chlor-1-cyclopropyl-6,7-difluor-1,4-dihydro-5-methyl-4-oxo-3-chinolin-carbonsäure und 3-Ethylaminomethyl-3-hydroxy-pyrrolidin als Ausgangsstoffe, so kann der Reaktionsablauf durch das folgende Formelschema wiedergegeben werden:

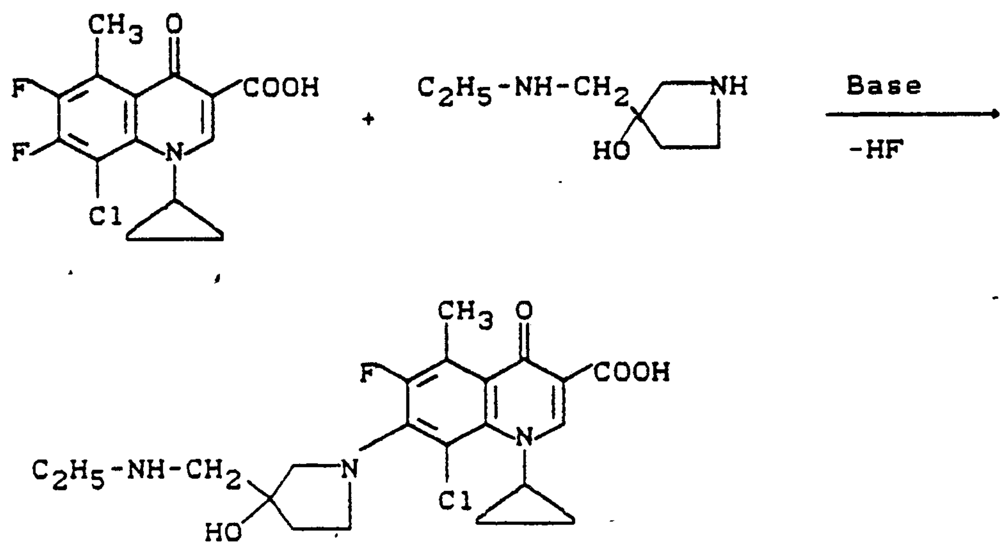


Verwendet man beispielsweise 1-Cyclopropyl-7-(2,7-diazabicyclo[3.3.0]oct-7-yl)-6-fluor-1,4-dihydro-5-methyl-4-oxo-3-chinolincarbonsäure und Ethanol/Chlorwasserstoff als Ausgangsstoffe, so kann der Reaktionsverlauf durch folgendes Formelschema wiedergegeben werden:

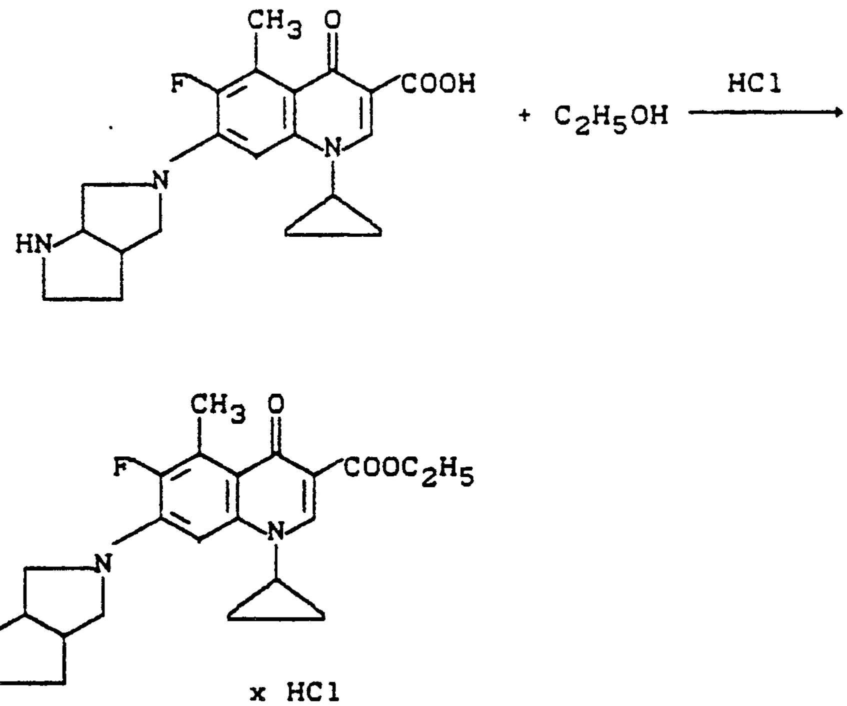


Die als Ausgangsstoffe verwendeten Verbindungen der Formel II sind bekannt oder können nach bekannten Methoden hergestellt werden. Als Beispiele seien genannt:
1-Cyclopropyl-6,7,8-trifluor-1,4-dihydro-5-methyl-4-oxo-3-chinolincarbonsäure,
8-Chlor-1-cyclopropyl-6,7-difluor-1,4-dihydro-5-methyl-4-oxo-3-chinolincarbonsäure,
1-Cyclopropyl-6,7-difluor-1,4-dihydro-5,8-dimethyl-4-oxo-3-chinolincarbonsäure,
1-Ethyl-6,7,8-trifluor-1,4-dihydro-5-methyl-4-oxo-3-chinolincarbonsäure,
1-Cyclopropyl-6,7,8-trifluor-1,4-dihydro-5-methyl-4-oxo-3-chinolincarbonsäureethylester,
8-Chlor-1-cyclopropyl-6,7-difluor-1,4-dihydro-5-methyl-4-oxo-3-chinolincarbonsäureethylester,
6,7-Difluor-1-(4-fluor-phenyl)-1,4-dihydro-5-methyl-4-oxo-3-chinolincarbonsäure,
6,7-Difluor-1-(2,4-difluor-phenyl)-1,4-dihydro-5-methyl-4-oxo-3-chinolincarbonsäure.

Die 7-Chlor-1-cyclopropyl-6,8-difluor-1,4-dihydro-5-methyl-4-oxo-3-chinolincarbonsäure ist nicht bekannt. Sie kann nach folgendem Schema dargestellt werden.

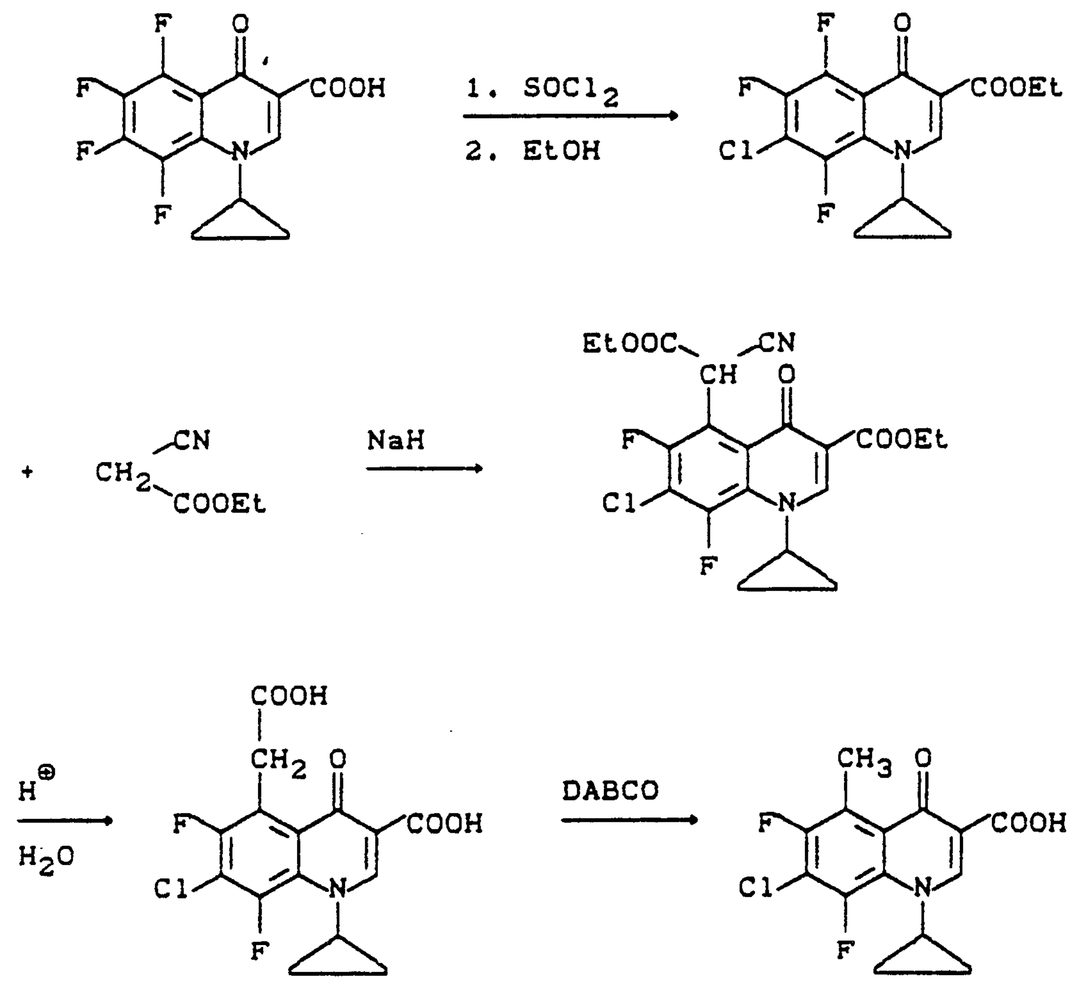


DABCO = 1,4-Diazabicyclo[2,2,2]octan

Die Verbindungen der Formel III mit den Strukturen

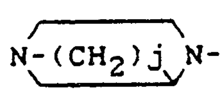 , 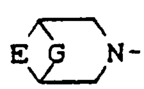 , 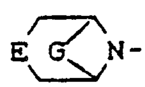 , 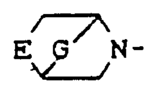 ,

sind bekannt (EP-PS 230 274).

Die als Ausgangsverbindungen verwendeten Verbindungen der Formel III mit den Strukturen

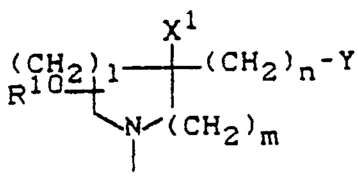

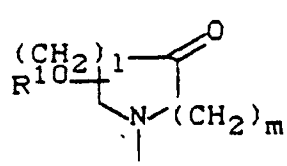

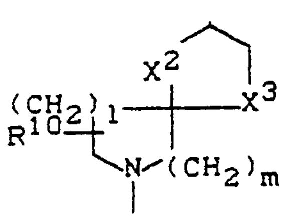 ,

sind zum teil neu.

Ihre Herstellung kann nach verschiedenen Verfahren erfolgen:

1. Durch Umsetzung der am Stickstoff geschützten Spiro-oxirane (1) [J. Med. Chem. 30, 222 (1987); US-P 4 508 724; EP-PS 189 370] mit Aminen (2) erfolgt Ringöffnung zu den Hydroxyaminen (3). Abspaltung der Schutzgruppe liefert Ausgangsverbindungen der Formel (IIIa):

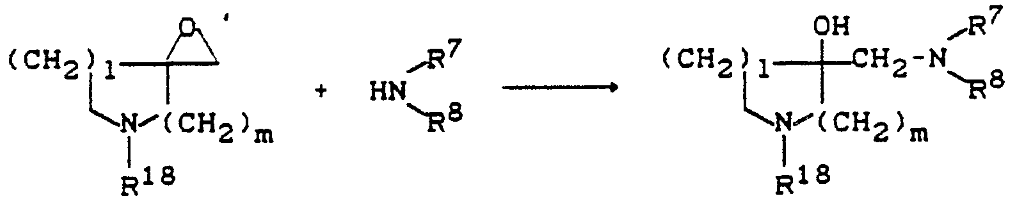

(1) (2) (3)

$R^{18}$ = COO-Alkyl, $CH_2C_6H_5$

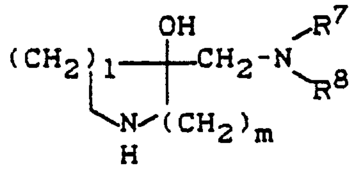

(IIIa)

2. Die Cyclisierung des Bernsteinsäureesters (4) [Tetrahedron Letters 46, 4561 (1973)] mit Benzylamin liefert den 1-Benzyl-3-hydroxy-5-oxo-pyrrolidin-3-carbonsäurealkylester (5), der nach Umsetzung mit einem Amin (2) zum Amid (6) reagiert. Nachfolgende Reduktion mit $LiAlH_4$ und hydrogenolytische Abspaltung der Benzylgruppe liefert Ausgangsverbindungen der Formel (IIIb):

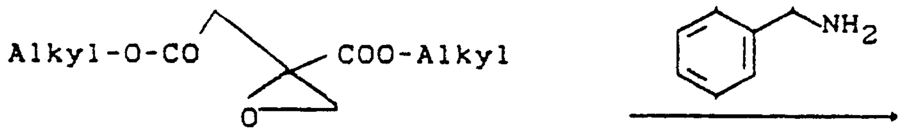

(4)

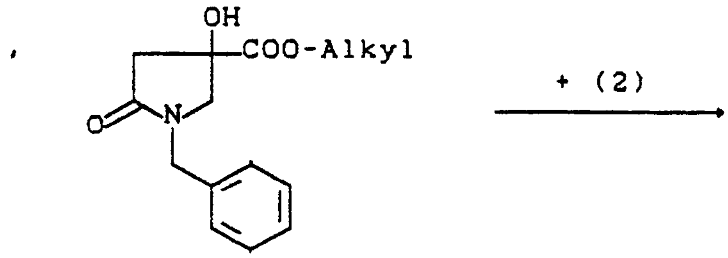


(5)

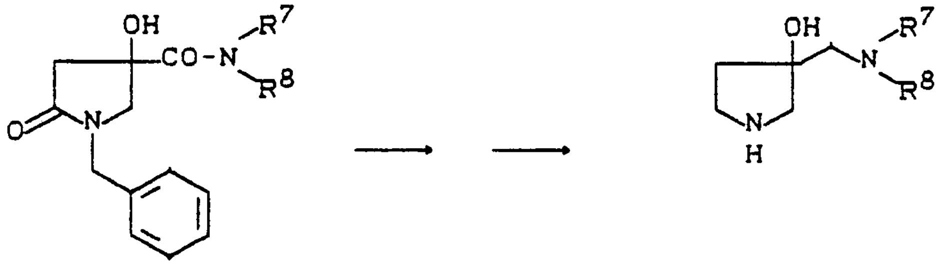

(6) (IIIb)

3. Umsetzung der (1-Benzyl-3-hydroxy-2,5-dioxo-pyrrolidin-3-yl)-essigsäure (7) [Gazz. Chim. Ital. 24, 226 (1894)] zum Amid (8) und nachfolgende Reduktion mit $LiAlH_4$ und Abspaltung der Benzylgruppe liefert Ausgangsverbindungen der Formel (IIIc):

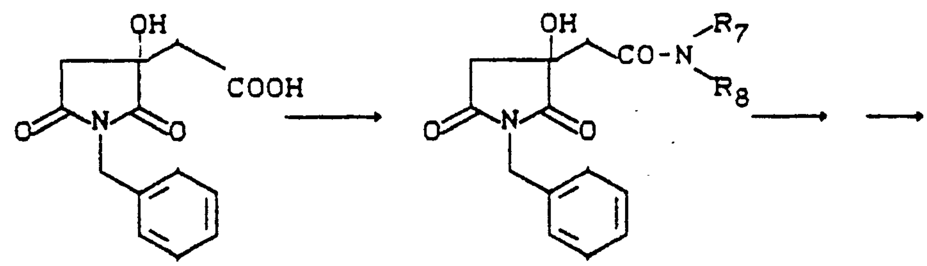

(7) (8)

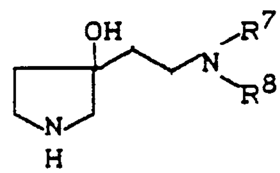

(IIIc)

4. 3-Hydroxy-3-methyl-pyrrolidin kann durch $LiAlH_4$-Reduktion von 4-Hydroxy-4-methyl-pyrrolidin-2-on [Zh. Org. Khim. 14, 7, S. 1420 (1978)] oder durch Debenzylierung von 1-Benzyl-3-hydroxy-3-methyl-pyrrolidin (EP 132 845) hergestellt werden.

5. Ausgehend von cyclischen Oxo-aminen (9), die am Stickstoff durch eine Schutzgruppe blockiert

sind, können Ausgangsverbindungen der Formeln (IIId), (IIIe), (IIIf) aufgebaut werden [Acta Chem. Scand. B 34, 319 (1980)].

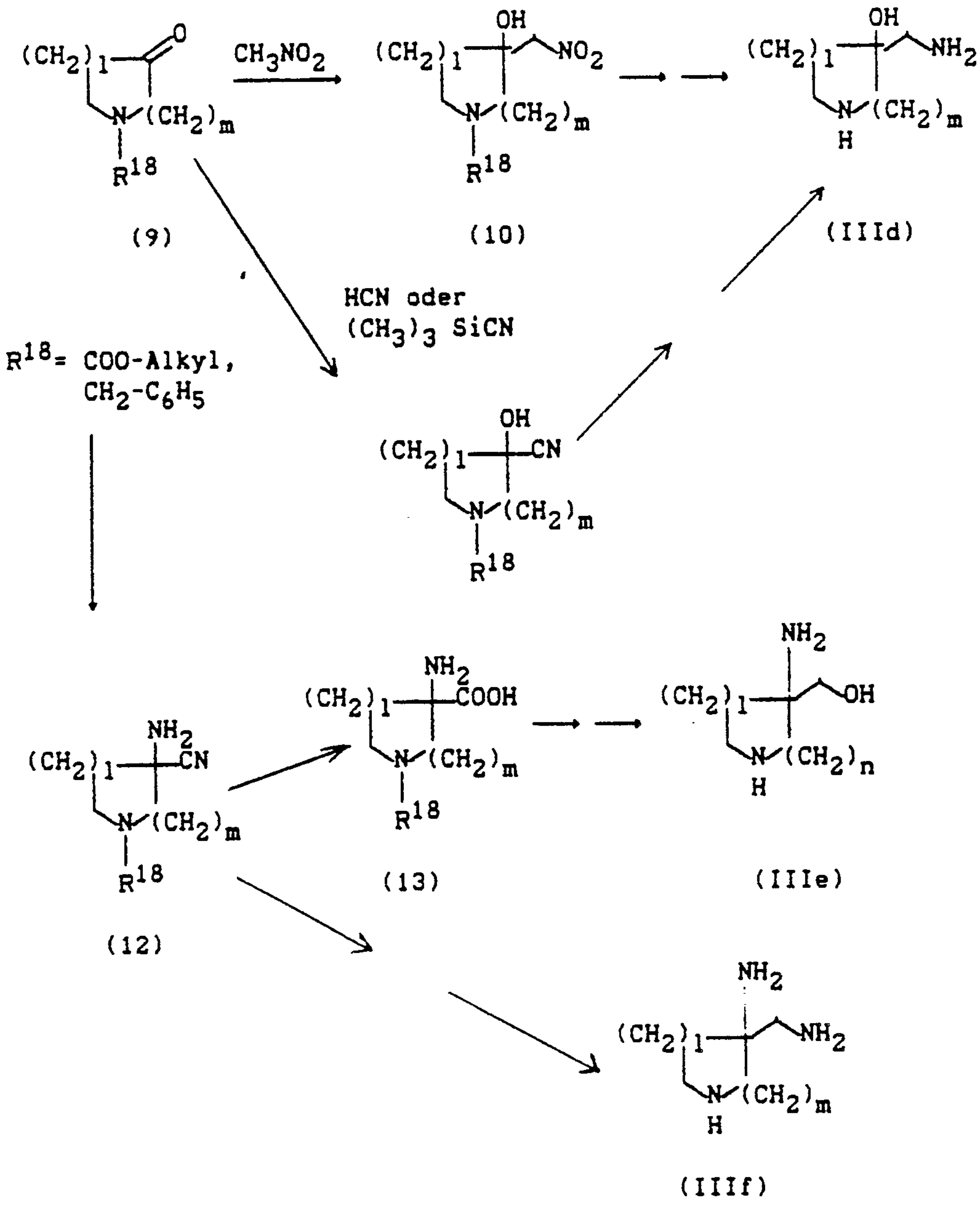


(9) (10) (IIId) (12) (13) (IIIe) (IIIf)

6. Die Hydroxygruppe der Hydroxyamine (IIIa) - (IIIe) kann alkyliert oder halogeniert werden.

7. Aus den cyclischen Oxoaminen (9) können Ketale, Thioketale oder Aminale hergestellt werden [Helv. Chim. Acta 50, 1289 (1967)].

Durch Umsetzung der am Stickstoff geschützten Spiro-oxirane (1) mit Trimethylsilylcyanid [J. Amer. Chem. Soc. 104, 5849 (1982)] können die Isonitrile (14) hergestellt werden, die durch Verseifen und Abspalten der Schutzgruppe zu den Ausgangsverbindungen der Formel (IIIg) umgesetzt werden können:

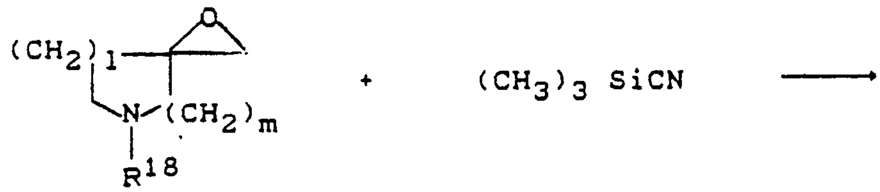

(1)

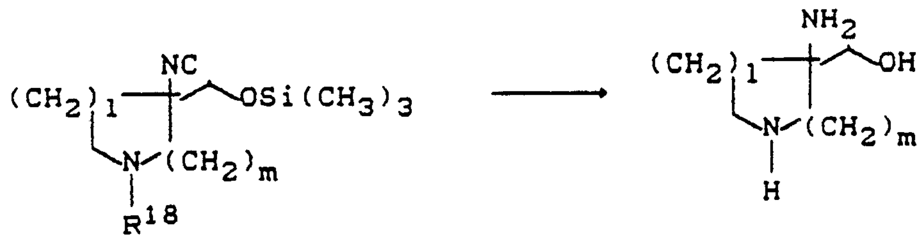

(14) (IIIg)

Als Beispiele für Ausgangsverbindungen der Formel (III) seien folgende Verbindungen genannt, wobei chirale Verbindungen sowohl als Racemate als auch als enantiomerenreine Stoffe eingesetzt werden können:

3-Aminomethyl-3-hydroxy-pyrrolidin,
3-Acetylaminomethyl-3-hydroxy-pyrrolidin,
3-tert.-Butoxycarbonylaminomethyl-3-hydroxy-pyrrolidin
3-Hydroxy-3-methylaminomethyl-pyrrolidin,
3-Ethylaminomethyl-3-hydroxy-pyrrolidin,
3-Hydroxy-3-propylaminomethyl-pyrrolidin,
3-Ethylaminomethyl-3-methoxy-pyrrolidin,
3-Ethoxy-3-ethylaminomethyl-pyrrolidin,
3-Chlor-3-ethylaminomethyl-pyrrolidin,
3-Ethylaminomethyl-3-fluor-pyrrolidin,
3-Ethylaminomethyl-3-methyl-pyrrolidin,
3-Ethylaminomethyl-3-mercapto-pyrrolidin,
3-Ethylaminomethyl-3-methylthio-pyrrolidin,
3-Acetoxy-3-ethylaminomethyl-pyrrolidin,
3-Dimethylaminomethyl-3-hydroxy-pyrrolidin,
3-Hydroxy-3-pyrrolidinomethyl-pyrrolidin,
3-Hydroxy-3-morpholinomethyl-pyrrolidin,
3-Amino-3-ethylaminomethyl-pyrrolidin,
3-Acetylamino-3-ethylaminomethyl-pyrrolidin,
3-Ethylaminomethyl-3-methylamino-pyrrolidin,
3-Dimethylamino-3-ethylaminomethyl-pyrrolidin,
3-Amino-3-hydroxymethyl-pyrrolidin,
3-Acetylamino-3-hydroxymethyl-pyrrolidin,
3-Amino-3-methoxymethyl-pyrrolidin,
3-tert.-Butoxycarbonylamino-3-methoxymethyl-pyrrolidin,
3-Amino-3-methylthiomethyl-pyrrolidin,
3-Amino-3-mercaptomethyl-pyrrolidin,
3-Cyclopropylaminomethyl-3-hydroxy-pyrrolidin,
3-Isopropylaminomethyl-3-hydroxy-pyrrolidin,
1,4-Dioxa-7-azaspiro[4.4]nonan,
1-Oxa-4,7-diazaspiro[4.4]nonan,
4-Methyl-1-oxa-4,7-diazaspiro[4.4]nonan,
1-Thia-4,7-diazaspiro[4.4]nonan,
1,4,7-Triazaspiro[4.4]nonan,
1,4-Dimethyl-1,4,7-triazaspiro[4.4]nonan.

Die als Ausgangsmaterial verwendeten Verbindungen der Formel III mit den Strukturen

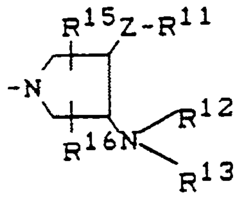 , 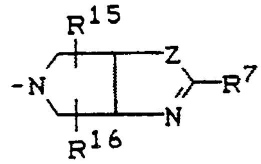 , 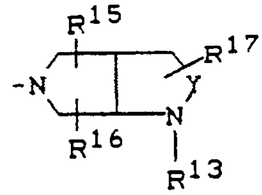

sind ebenfalls zum Teil neu. Sie können nach folgenden Verfahren hergestellt werden.

1. Ausgehend von dem N-geschützten 3,4-Epoxypyrrolidin (1) (Deutsche Offenlegungsschrift 1 929 237, US-Patent 4 254 135), das gegebenenfalls noch eine oder zwei Methyl- oder Phenylreste tragen kann, werden die Ausgangsverbindungen der Formel (IIIa)-(IIIe) hergestellt.

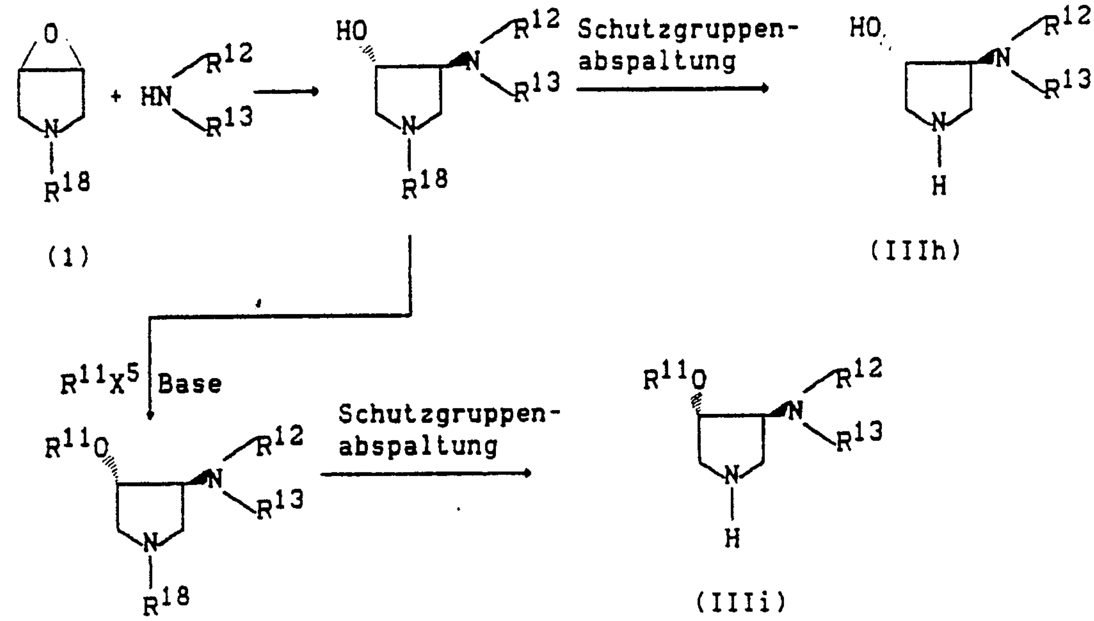


(1) (IIIh) (IIIi)

$R^{18}$ = Benzyl, Acyl, Alkoxycarbonyl, Benzyloxycarbonyl, Trialkylsilyl, Sulfonyl (Beispiele für Schutzgruppen),

X = Abgangsgruppe wie Halogen, Alkyl- oder Arylsulfonyloxy

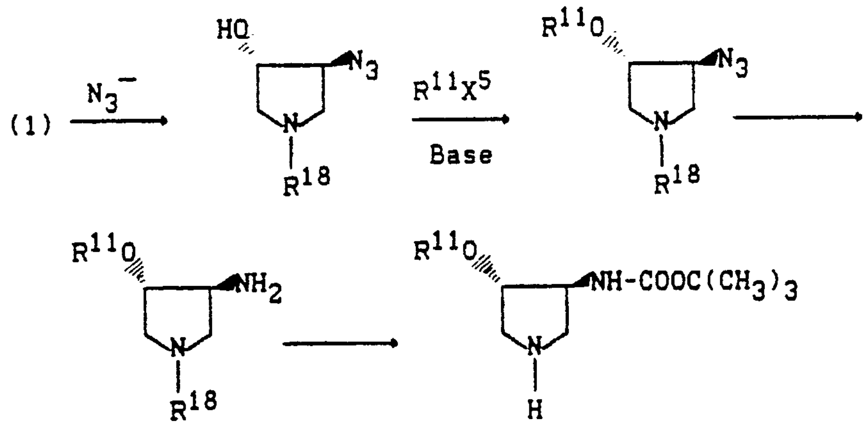


(1)

(IIIj)

(1)

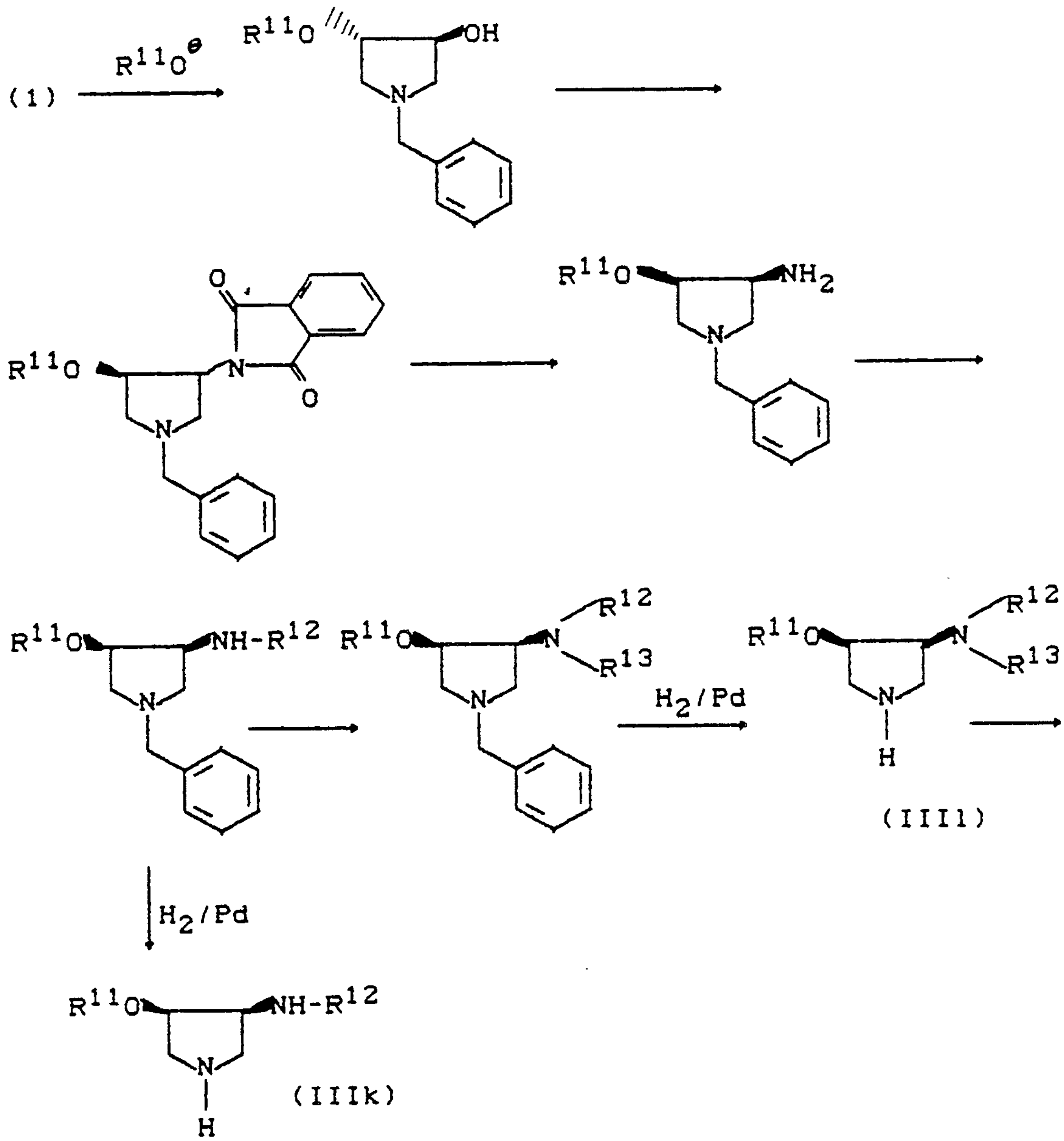


(IIIl)

(IIIk)

2. Ausgangsverbindungen der Formel (IIIm) erhält man aus 2-(1,2-Dichlorethyl)-oxiran über die folgende Reaktionssequenz:

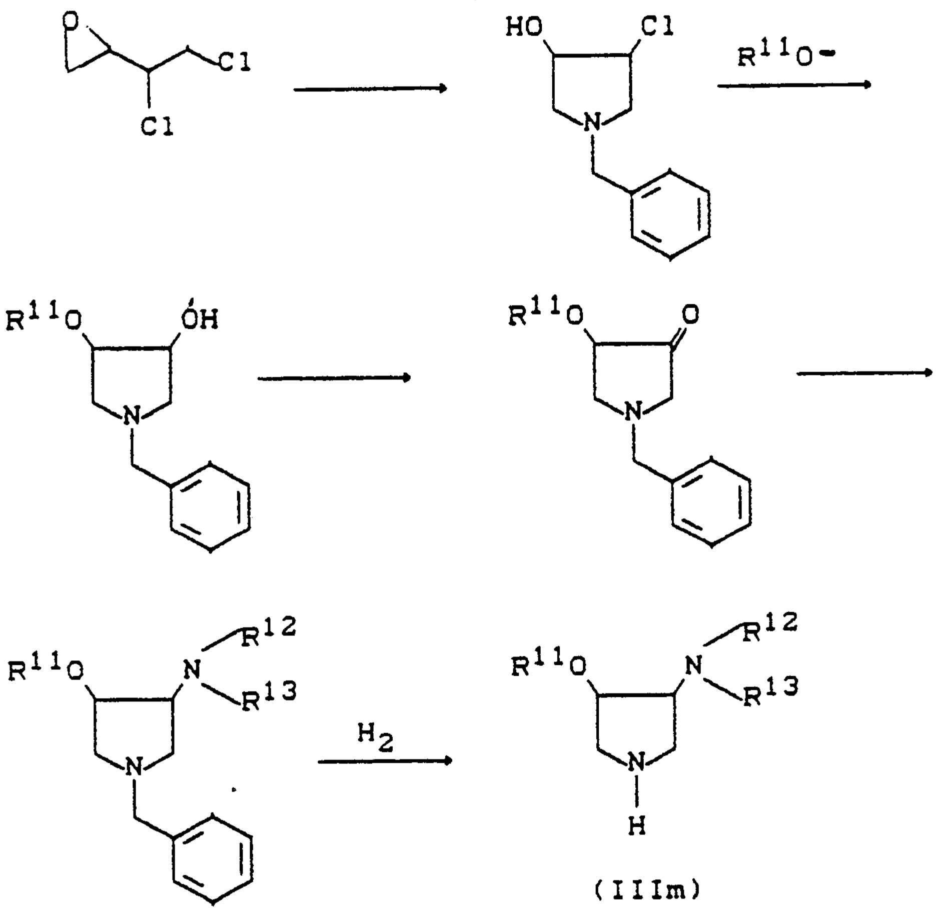


(IIIm)

3. Durch Addition von Aziden an gegebenenfalls mit einem oder zwei Methyl- beziehungsweise Phenylresten substituierte N-Benzylmaleinimide können Ausgangsverbindungen der Formel (III n) hergestellt werden:

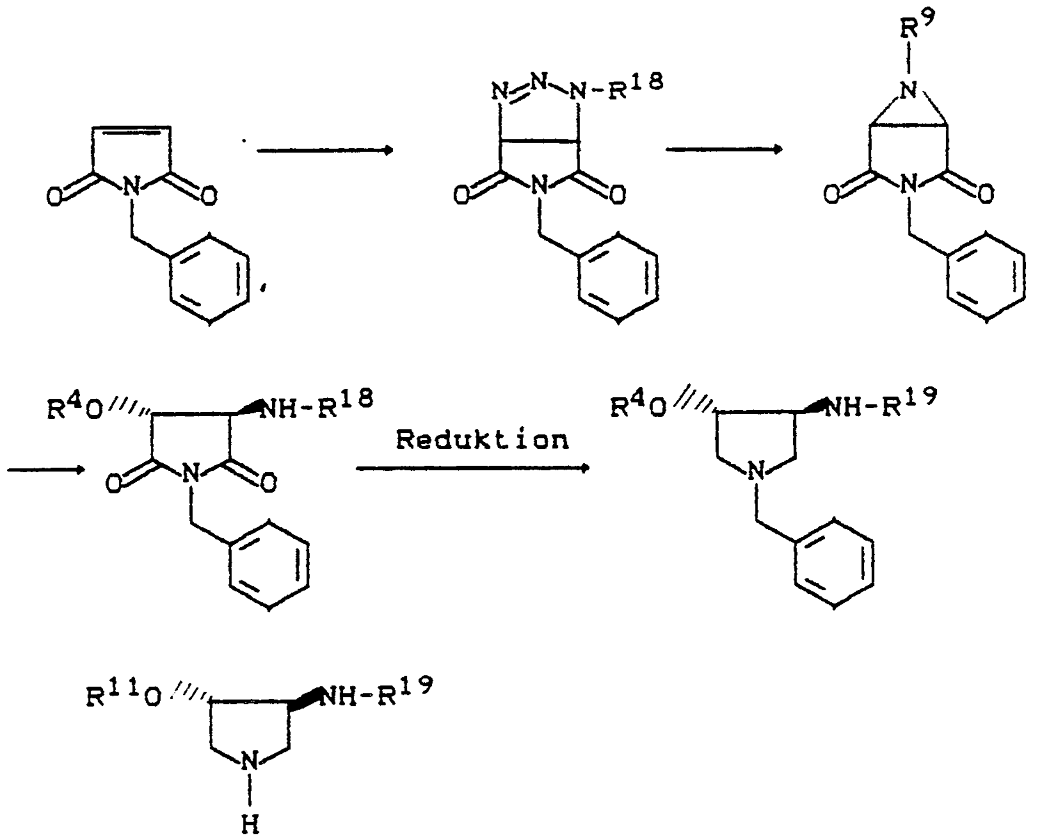


(IIIn)

$R^{19}$ = H, Alkyl, Benzyl.

4. Aus den 3,4-Epoxypyrrolidinen (1) erhält man über eine Cyclisierung mit Thionylchlorid die Ausgangsverbindungen der Formel (III o):

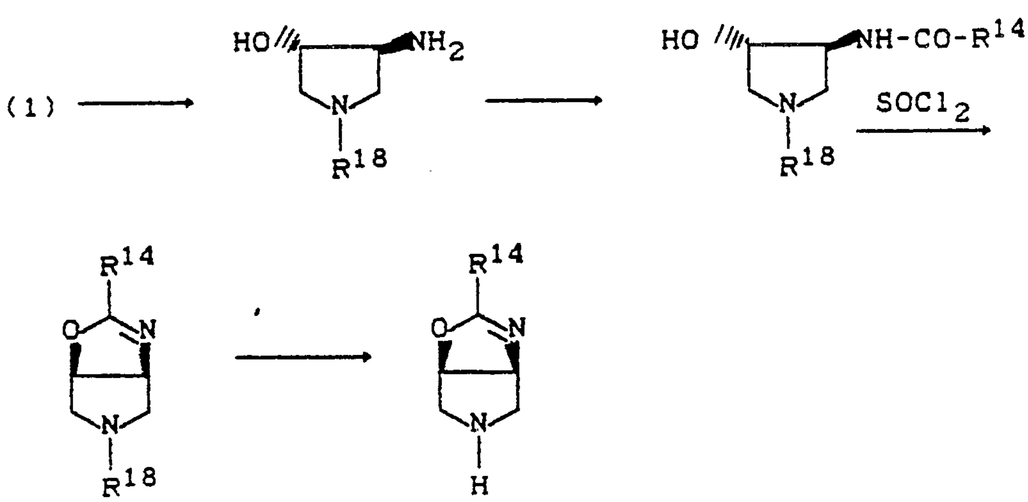


(III o)

5. Durch Umsetzen der 3,4-Epoxypyrrolidine (1) mit Ethanolaminen erhält man durch intramolekulare Veretherung die Ausgangsverbindungen der Formel (III p):

(1)

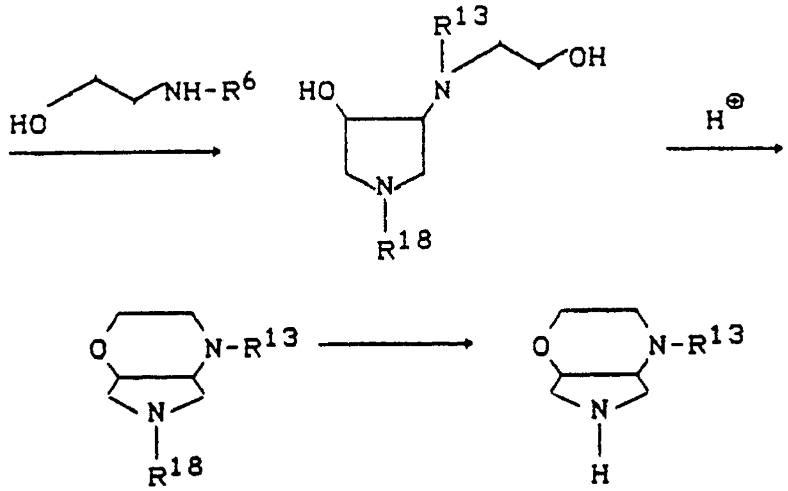


(III p).

6. Die Ausgangsverbindungen der Formel (III q) erhält man aus Aminoacetaldehyddimethylacetal über eine intramolekulare 1,3-dipolare Cycloaddition.

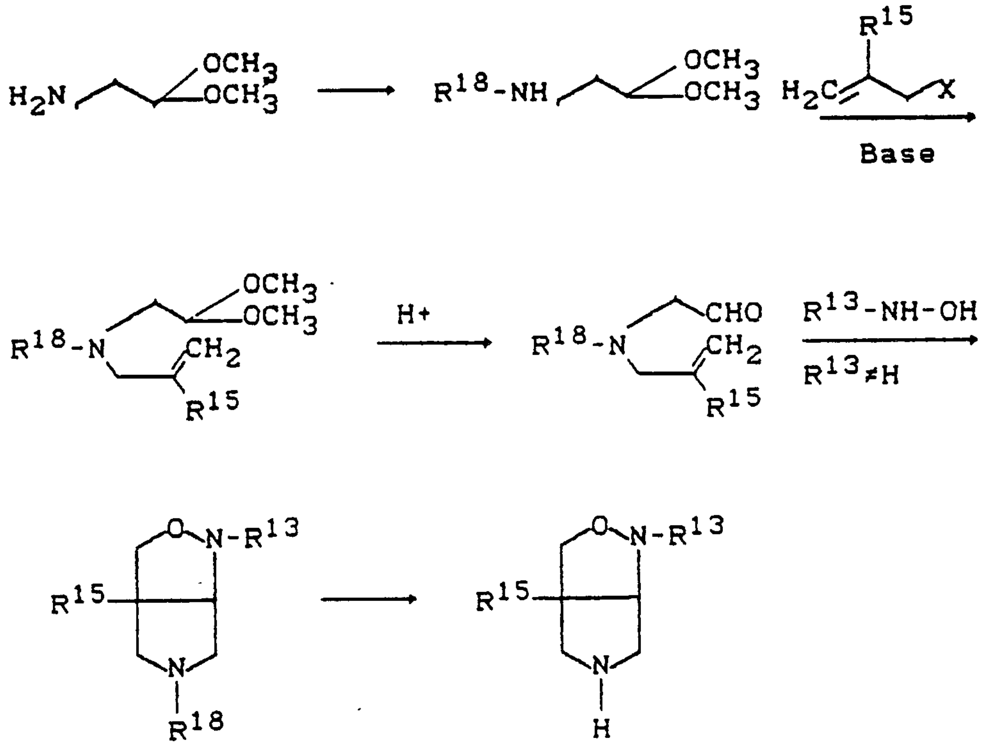


(III q)

7. Ausgehend von Pyridin-2,3-dicarbonsäure-N-benzylimid werden Ausgangsverbindungen (III r) beziehungsweise (III l) über die angegebenen Reaktionsschritte hergestellt.

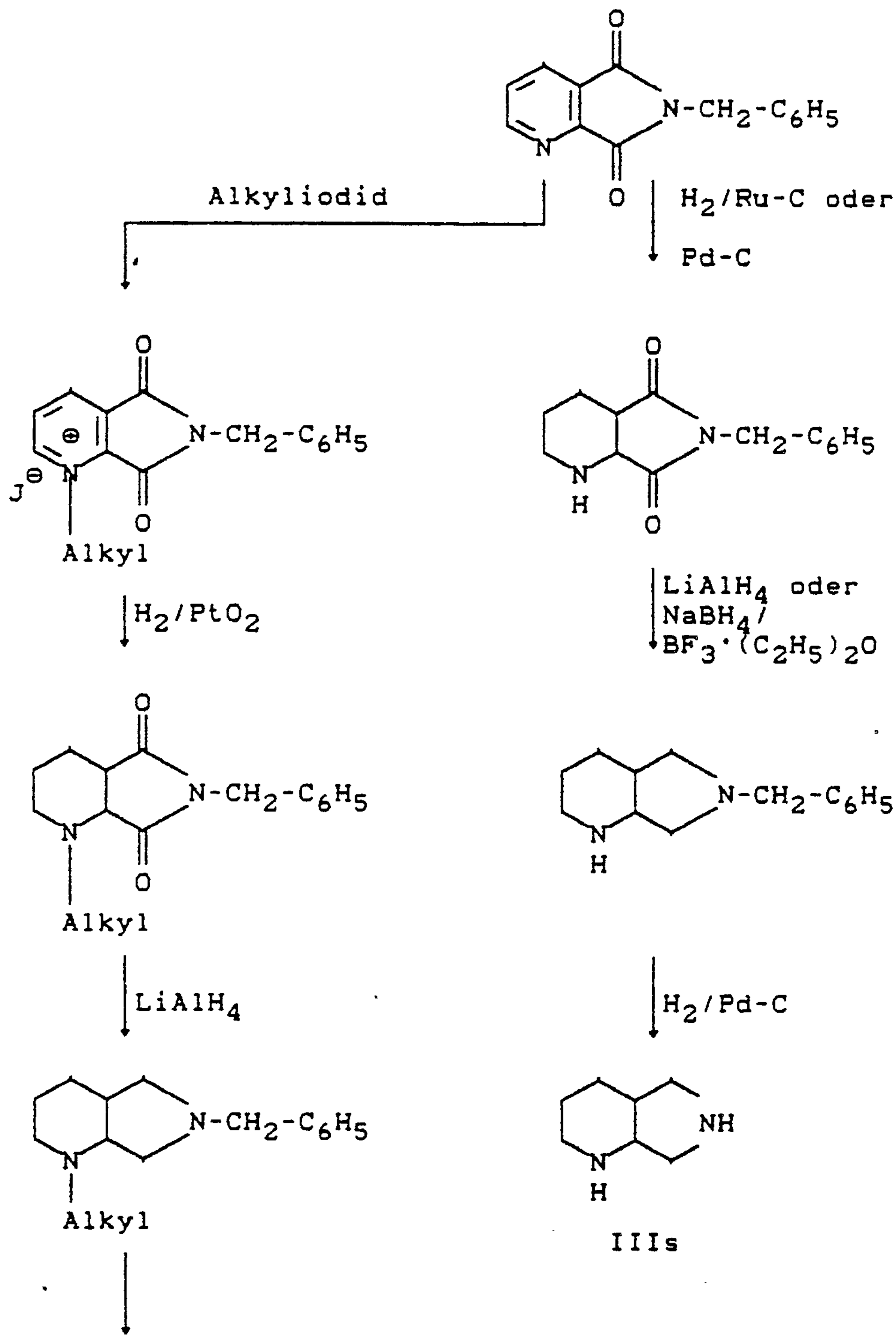
Alkyliodid
H2/Ru-C oder
Pd-C
H2/PtO2
LiAlH4 oder
NaBH4/
BF3·(C2H5)2O
LiAlH4
H2/Pd-C
IIIs

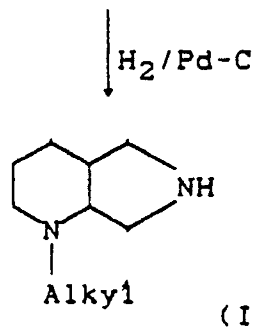


(III r)

8. N-Benzyl-maleinsäureimid addiert 2-Chlorethylamine zu den 3-(2-chlorethylamino)-succinimiden, die zu den Ausgangsverbindungen der Formel (III t) umgesetzt werden:

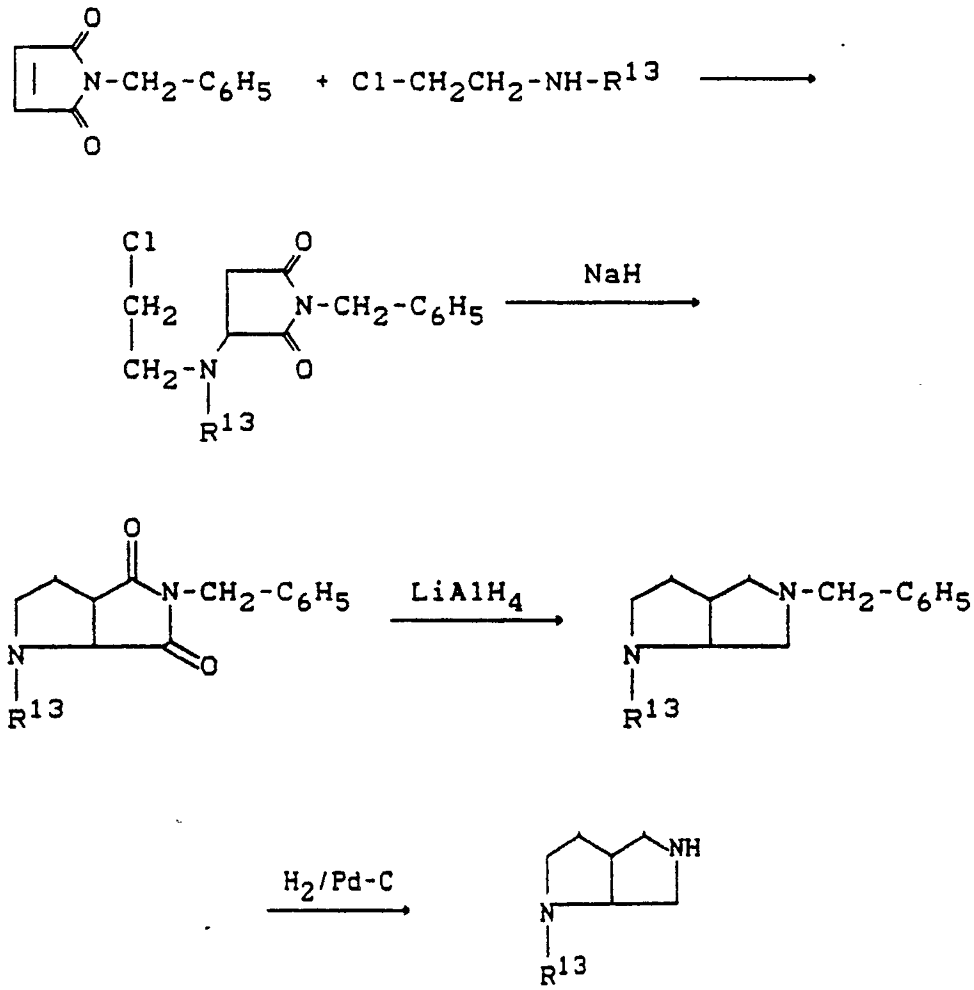


(III t)

9. 2-Methyl-2-propenal-dimethylhydrazon reagiert mit N-Benzylmaleinsäureimid zu einem Cycloaddukt, welches nach der angegebenen Reaktionsfolge in die Ausgangsverbindung (III u) überführt werden kann.

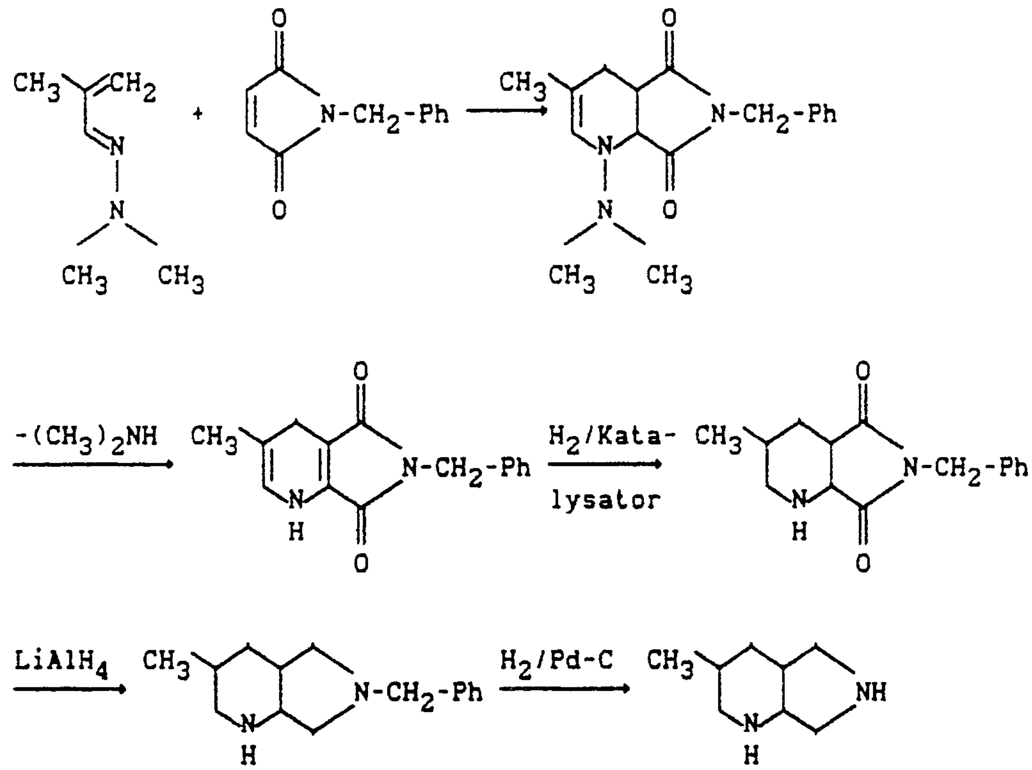


(III u)

Nach diesem allgemeinen Formelschema lassen sich zum Beispiel die folgenden Ausgangsverbindungen herstellen. Sie können als Diastereomerengemische, in diastereomerenreiner und auch enantiomerenreiner Form hergestellt und eingesetzt werden.

4-Amino-3-hydroxypyrrolidin,
3-Hydroxy-4-methylaminopyrrolidin,
4-Dimethylamino-3-hydroxypyrrolidin,
4-Ethylamino-3-hydroxypyrrolidin,
3-Amino-4-methoxypyrrolidin,
4-Methoxy-3-methylaminopyrrolidin,
3-Dimethylamino-4-methoxypyrrolidin,
3-Ethylamino-4-methoxypyrrolidin,
3-Amino-4-ethoxypyrrolidin,
4-Ethoxy-3-methylaminopyrrolidin,
3-Dimethylamino-4-ethoxypyrrolidin,
4-Ethoxy-3-ethylaminopyrrolidin,
3-Hydroxy-4-hydroxyaminopyrrolidin,
3-Hydroxy-4-methoxyaminopyrrolidin,
3-Hydroxyamino-4-methoxypyrrolidin,
4-Methoxy-3-methoxyaminopyrrolidin,
3-Benzylamino-4-methoxypyrrolidin,
4-Methoxy-3-((5-methyl-2-oxo-1,3-dioxol-4-yl)-methylamino)-pyrrolidin,
3-Amino-4-methylmercaptopyrrolidin,
3-Acetoxy-4-dimethylaminopyrrolidin,
3-Acetamido-4-methoxypyrrolidin,
4-Methoxy-3-methoxycarbonylaminopyrrolidin,
3-Formamido-4-methoxypyrrolidin,
3-Amino-4-methoxy-2-methylpyrrolidin,
3-Amino-4-methoxy-5-methylpyrrolidin,
4-Methoxy-2-methyl-3-methylaminopyrrolidin,
4-Methoxy-5-methyl-3-methylaminopyrrolidin,
3-Amino-4-methoxy-2-phenylpyrrolidin,

4-Methoxy-3-methylamino-5-phenylpyrrolidin,
3-Methyl-2,7-diazabicyclo[3.3.0]octan,
4-Methyl-2,7-diazabicyclo[3.3.0]octan,
5-Methyl-2,7-diazabicyclo[3.3.0]octan,
3,5-Dimethyl-2,7-diazabicyclo[3.3.0]octan,
1,5-Dimethyl-2,7-diazabicyclo[3.3.0]octan,
2-Oxa-4,7-diazabicyclo[3.3.0]octan,
3,3-Dimethyl-2-oxa-4,7-diazabicyclo[3.3.0]octan,
3-Oxa-2,7-diazabicyclo[3.3.0]octan,
1,2-Dimethyl-3-oxa-2,7-diazabicyclo[3.3.0]octan,
2,5-Dimethyl-3-oxa-2,7-diazabicyclo[3.3.0]octan,
2,8-Dimethyl-3-oxa-2,7-diazabicyclo[3.3.0]octan,
5-Methyl-3-oxa-2,7-diazabicyclo[3.3.0]octan,
2-Oxa-4,7-diazabicyclo[3.3.0]oct-3-en,
3-Methyl-2-oxa-4,7-diazabicyclo[3.3.0]oct-3-en,
3-Phenyl-2-oxa-4,7-diazabicyclo[3.3.0]oct-3-en,
6-Methyl-2-oxa-4,7-diazabicyclo[3.3.0]oct-3-en,
8-Methyl-2-oxa-4,7-diazabicyclo[3.3.0]oct-3-en,
3-Methyl-2,8-diazabicyclo[4.3.0]nonan,
4-Methyl-2,8-diazabicyclo[4.3.0]nonan,
5-Methyl-2,8-diazabicyclo[4.3.0]nonan,
6-Methyl-2,8-diazabicyclo[4.3.0]nonan,
3-Methyl-2-oxa-5,8-diazabicyclo[4.3.0]nonan,
4-Methyl-2-oxa-5,8-diazabicyclo[4.3.0]nonan,
1-Methyl-2-oxa-5,8-diazabicyclo[4.3.0]nonan,
3,5-Dimethyl-2-oxa-5,8-diazabicyclo[4.3.0]nonan,
2-Thia-5,8-diazabicyclo[4.3.0]nonan,
5-Methyl-2-thia-5,8-diazabicyclo[4.3.0]nonan,
3,5-Dimethyl-2-thia-5,8-diazabicyclo[4.3.0]nonan,
3-Oxa-2,8-diazabicyclo[4.3.0]nonan,
2-Methyl-9-oxa-2,8-diazabicyclo[4.3.0]nonan,
4-Methyl-3-oxa-2,8-diazabicyclo[4.3.0]nonan,
2,5-Dimethyl-3-oxa-2,8-diazabicyclo[4.3.0]nonan,
3-Oxa-5,8-diazabicyclo[4.3.0]nonan,
5-Methyl-3-oxa-5,8-diazabicyclo[4.3.0]nonan,
1,5-Dimethyl-3-oxa-5,8-diazabicyclo[4.3.0]nonan,
4,4-Dimethyl-3-oxa-5,8-diazabicyclo[4.3.0]nonan.

Die Umsetzung von (II) mit (III), bei der die Verbindungen (III) auch in Form ihrer Hydrochloride eingesetzt werden können, wird vorzugsweise in einem Verdünnungsmittel wie Dimethylsulfoxid, N,N-Dimethylformamid, N-Methylpyrrolidon, Hexamethyl-phosphorsäuretrisamid, Sulfolan, Acetonitril, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo[2.2.2]octan (DABCO), 1,8-Diazabicyclo[5,4,0]undec-7-en (DBU) oder überschüssiges Amin (III).

Die Reaktionstemperaturen können in einem größeren Bereich variiert werden. Im allgemeinen arbeitet man zwischen etwa 20 und 200 °C, vorzugsweise zwischen 80 und 180 °C.

Die Umsetzung kann bei Normaldruck, aber auch bei erhöhtem Druck durchgeführt werden. Im allgemeinen arbeitet man bei Drucken zwischen etwa 1 und 100 bar, vorzugsweise zwischen 1 und 10 bar.

Bei der Durchführung des erfindungsgemäßen Verfahrens setzt man auf 1 Mol der Carbonsäure (II) 1 bis 15 Mol, vorzugsweise 1 bis 6 Mol der Verbindung (III) ein.

Freie Hydroxygruppen können während der Umsetzung durch eine geeignete Hydroxyschutzgruppe, zum Beispiel durch den Tetrahydropyranylrest, geschützt und nach Beendigung der Reaktion wieder freigesetzt werden (siehe J.F.W. McOmie, Protective Groups in Organic Chemistry (1973), Seite 104).

Freie Aminofunktionen können während der Umsetzung durch eine geeignete Aminoschutzgruppe, zum Beispiel durch den Ethoxycarbonyl- oder den tert.-Butoxycarbonylrest, geschützt und nach Beendigung der Reaktion durch Behandlung mit einer geeigneten Säure wie Chlorwasserstoffsäure oder Trifluoresigsäure

wieder freigesetzt werden (siehe Houben-Weyl, Methoden der oganischen Chemie, Band E4, Seite 144 (1983); J.F.W. McOmie, Protective Groups in Organic Chemistry (1973), Seite 43).

Zur Herstellung der erfindungsgemäßen Ester wird die zugrundeliegende Carbonsäure vorzugsweise in überschüssigem Alkohol in Gegenwart von starken Säuren, wie Schwefelsäure, wasserfreiem Chlorwasserstoff, Methansulfonsäure, p-Toluolsulfonsäure oder sauren Ionenaustauschern, bei Temperaturen von etwa 20° bis 200° C, vorzugsweise etwa 60° bis 120° C umgesetzt. Das entstehende Reaktionswasser kann auch durch azeotrope Destillation mit Chloroform, Tetrachlormethan, Benzol oder Toluol entfernt werden.

Die Herstellung von Estern gelingt auch vorteilhaft durch Erhitzen der zugrundeliegenden Säure mit Dimethylformamiddialkylacetal in einem Lösungsmittel wie Dimethylformamid.

Die als Prodrug verwendeten (5-Methyl-2-oxo-1,3-dioxol-4 yl)-methylester werden durch Umsetzung eines Alkalisalzes der zugrundeliegenden Carbonsäure mit 4-Brommethyl- oder 4-Chlormethyl-5-methyl-1,3-dioxol-2-on in einem Lösungsmittel wie Dimethylformamid, Dimethylacetamid, N-Methylpyrrolidon, Dimethylsulfoxid oder Tetramethylharnstoff bei Temperaturen von etwa 0° bis 100° C, vorzugsweise 0° bis 50° C, erhalten.

Die Herstellung der Säureadditionssalze der erfindungsgemäßen Verbindungen erfolgt in üblicher Weise zum Beispiel durch Lösen des Betains in überschüssiger wäßriger Säure und Ausfällen des Salzes mit einem mit Wasser mischbaren organischen Lösungsmittel wie Methanol, Ethanol, Aceton, Acetonitril. Man kann auch äquivalente Mengen Betain und Säure in Wasser oder einem Alkohol wie Glykolmonomethylether erhitzen und anschließend bis zur Trockne eindampfen oder das ausgefallene Salz absaugen. Als pharmazeutisch verwendbare Salze sind beispielsweise die Salze von Salzsäure, Schwefelsäure, Essigsäure, Glykolsäure, Milchsäure, Bernsteinsäure, Zitronensäure, Weinsäure, Methansulfonsäure, 4-Toluolsulfonsäure, Galacturonsäure, Gluconsäure, Embonsäure, Glutaminsäure oder Asparaginsäure zu verstehen.

Die Alkali- oder Erdalkalisalze der erfindungsgemäßen Carbonsäuren werden beispielsweise durch Lösen des Betains in unterschüssiger Alkali- oder Erdalkalilauge, Filtration von ungelöstem Betain und Eindampfen des Filtrats bis zur Trockne erhalten. Pharmazeutisch geeignet sind Natrium-, Kalium- oder Calciumsalze. Durch Umsetzung eines Alkali- oder Erdalkalisalzes mit einem geeigneten Silbersalz wie Silbernitrat werden die entsprechenden Silbersalze erhalten.

Außer den in den Beispielen genannten Wirkstoffen können ebenfalls die in Tabelle 1 beispielhaft aufgeführten Verbindungen hergestellt werden, wobei diese Verbindungen sowohl als Diastereomerengemische als auch als diastereomerenreine oder enantiomerenreine Verbindungen vorliegen können.

Die erfindungsgemäßen Verbindungen zeigen bei geringer Toxizität ein breites antibakterielles Spektrum gegen gram-positive und gram-negative Keime, insbesondere gegen Enterobakteriaceen; vor allem auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gram-negative und gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden: Gram-positive Kokken, z.B. Staphylokokken (Staph. aureus, Staph. epidermidis) und Streptokokken (Strept. agalactiae, Strept. faecalis, Strept. pneumoniae, Strept. pyogenes); gram-negative Kokken (Neisseria gonorrhoeae) sowie gram-negative Stäbchen wie Enterobakteriaceen, z.B. Escherichia coli, Haemophilus influenzae, Citrobacter (Citrob. freundii, Citrob. divernis), Salmonella und Shigella; ferner Klebsiellen (Klebs. pneumoniae, Klebs. oxytoca), Enterobacter (Ent. aerogenes, Ent. agglomerans), Hafnia, Serratia (Serr. marcescens), Proteus (Pr. mirabilis, Pr. rettgeri, Pr. vulgaris), Providencia, Yersinia, sowie die Gattung Acinetobacter. Darüber hinaus umfaßt das antibakterielle Spektrum die Gattung Pseudomonas (Ps. aeruginosa, Ps. maltophilia) sowie strikt anaerobe Bakterien wie z.B. Bacteroides fragilis, Vertreter der Gattung Peptococcus, Peptostreptococcus sowie die Gattung Clostridium; ferner Mykoplasmen (M. pneumoniae, M. hominis, M. urealyticum) sowie Mykobakterien, z.B. Mycobacterium tuberculosis.

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen Verbindungen verhindert, gebessert oder geheilt werden können, seien beispielsweise genannt:
Infektionskrankheiten beim Menschen wie zum Beispiel Otitis, Pharyngitis, Pneumonie, Peritonitis, Pyelonephritis, Cystitis, Endocarditis, Systeminfektionen, Bronchitis (akut, chronisch), septische Infektionen, Erkrankungen der oberen Luftwege, diffuse Panbronchiolitis, pulmonäres Emphysem, Dysenterie, Enteritis, Leberabszesse, Urethritis, Prostatitis, Epididymitis, gastrointestinale Infektionen, Knochen- und Gelenkinfektionen, zystische Fibrose, Hautinfektionen, postoperative Wundinfektionen, Abszesse, Phlegmone, Wundinfektionen, infizierte Verbrennungen, Brandwunden, Infektionen im Mundbereich, Infektionen nach Zahnoperationen, Osteomyelitis, septische Arthritis, Cholecystitis, Peritonitis mit Appendicitis, Cholangitis, intraabdominale Abszesse, Pankreatitis, Sinusitis, Mastoiditis, Mastitis, Tonsillitis, Typhus, Meningitis und Infektionen des Nervensystems, Salpingitis, Endometritis, Genital-Infektionen, Pelveoperitonitis und Augeninfektionen.

Außer beim Menschen können bakterielle Infektionen auch bei anderen Spezies behandelt werden. Beispielhaft seien genannt:
Schwein: Coli-diarrhoe, Enterotoxämie, Sepsis, Dysenterie, Salmonellose, Mastitis-Metritis-Agalaktie-Syndrom, Mastitis;
Wiederkäuer (Rind, Schaf, Ziege): Diarrhoe, Sepsis, Bronchopneumonie, Salmonellose, Pasteurellose, Mykoplasmose, Genitalinfektionen;
Pferd: Bronchopneumonien, Fohlenlähme, puerperale und postpuerperale Infektionen, Salmonellose;
Hund und Katze: Bronchopneumonie, Diarrhoe, Dermatitis, Otitis, Harnwegsinfekte, Prostatitis;
Geflügel (Huhn, Pute, Wachtel, Taube, Ziervögel und andere): Mycoplasmose, E. coli-Infektionen, chronische Luftwegserkrankungen, Salmonellose, Pasteurellose, Psittakose.

Ebenso können bakterielle Erkrankungen bei der Aufzucht und Haltung von Nutz- und Zierfischen behandelt werden, wobei sich das antibakterielle Spektrum über die vorher genannten Erreger hinaus auf weitere Erreger wie zum Beispiel Pasteurella, Brucella, Campylobacter, Listeria, Erysipelothrix, Corynebakterien, Borrelia, Treponema, Nocardia, Rickettsien, Yersinia, erweitert.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetyl-alkohol, Glycerinmonostearat, (h) Adsorptionsmittel, z.B. Kaolin und Bentonit und (i) Gleitmittel z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbesserte Zusätze, z.B. Pfefferminzöl und Eukalyptusöl und Süßmittel, z.B Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracisternal, intravaginal, intraperitoneal, lokal (Puder, Salbe, Tropfen) und zur Therapie von Infektionen in Hohlräumen, Körperhöhlen angewendet werden. Als geeignete Zubereitungen kommen Injektionlösungen, Lösungen und Suspensionen für die orale Therapie, Gele, Aufgußformulierungen, Emulsionen, Salben oder Tropfen in Frage. Zur lokalen Therapie können ophthalmologische und dermatologische Formu lierungen, Silber- und andere Salze, Ohrentropfen, Augensalben, Puder oder Lösungen verwendet werden. Bei Tieren kann die Aufnahme auch über das Futter oder Trinkwasser in geeigneten Formulierungen erfolgen. Ferner können Gele, Pulver, Puder, Tabletten, Retard-Tabletten, Premixe, Konzentrate, Granulate, Pellets, Boli, Kapseln, Aerosole, Sprays, Inhalate bei Mensch und Tier angewendet werden. Ferner können die erfindungsgemäßen Verbindungen in andere Trägermaterialien wie zum Beispiel Kunststoffe, (Kunststoffketten zur lokalen Therapie), Kollagen oder Knochenzement eingearbeitet werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen können in den üblichen Konzentrationen und Zubereitungen zusammen mit

dem Futter bzw. mit Futterzubereitungen oder mit dem Trinkwasser gegeben werden. Dadurch kann eine Infektion durch gram-negative oder gram-positive Bakterien verhindert, gebessert und/oder geheilt werden und dadurch eine Förderung des Wachstums und eine Verbesserung der Verwertung des Futters erreicht werden.

Die minimalen Hemmkonzentrationen (MHK) wurden per Reihenverdünnungsverfahren auf Iso-Sensitest Agar (Oxoid) bestimmt. Für jede Prüfsubstanz wurde eine Reihe von Agarplatten hergestellt, die bei jeweils doppelter Verdünnung abfallende Konzentrationen des Wirkstoffes enthielten. Die Agarplatten wurden mit einem Multipoint-Inokulator (Denley) beimpft. Zum Beimpfen wurden Übernachtkulturen der Erreger verwandt, die zuvor so verdünnt wurden, daß jeder Impfpunkt ca. $10^4$ koloniebildende Partikel enthielt. Die beimpften Agarplatten wurden bei 37 °C bebrütet, und das Keimwachstum wurde nach ca. 20 Stunden abgelesen. Der MHK-Wert (µg/ml) gibt die niedrigste Wirkstoffkonzentration an, bei der mit bloßem Auge kein Keimwachstum zu erkennen war.

<u>**Tabelle 1**</u>

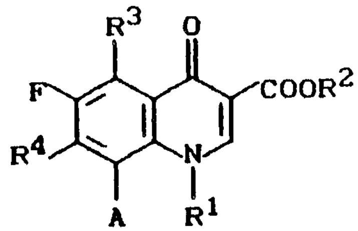

| $R^1$ | $R^2$ | $R^4$ | $R^3$ | A |
|---|---|---|---|---|
| 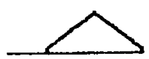 | H | 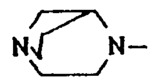 | $CH_3$ | H |
| 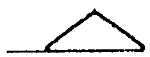 | $C_2H_5$ | 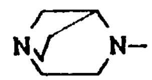 | $CH_3$ | H |
| 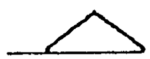 | H | 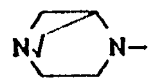 | $CH_3$ | F |
| 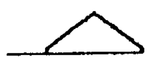 | H | 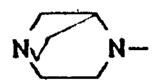 | $CH_3$ | Cl |

Tabelle 1 (Fortsetzung)

| $R^1$ | $R^2$ | $R^4$ | $R^3$ | A |
|---|---|---|---|---|
| $-C_2H_5$ | H | 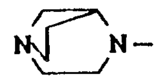 | $CH_3$ | H |
| $-C_2H_5$ | H | 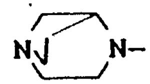 | $CH_3$ | F |
| 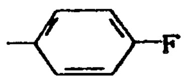 | H | 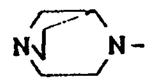 | $CH_3$ | H |
| 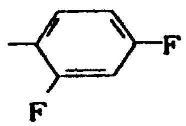 | H | 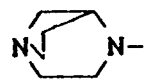 | $CH_3$ | H |
| $-CH=CH_2$ | H | 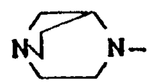 | $CH_3$ | H |
| $HO-CH_2CH_2-$ | H | 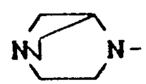 | $CH_3$ | H |
| 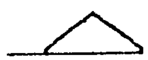 | H | 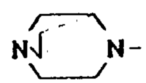 | $C_2H_5$ | H |

<u>Tabelle 1</u> (Fortsetzung)

| $R^1$ | $R^2$ | $R^4$ | $R^3$ | A |
|---|---|---|---|---|
| 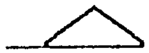 | H | 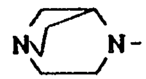 | $C_2H_5$ | F |
| 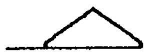 | $-C_2H_5$ | 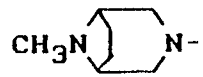 | $CH_3$ | H |
| 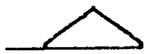 | 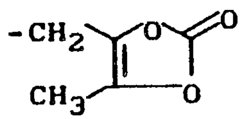 | 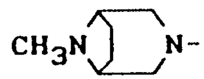 | $CH_3$ | F |
| 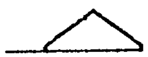 | H | 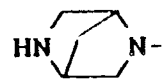 | $CH_3$ | H |
| 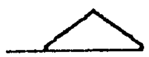 | H | 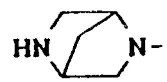 | $C_2H_5$ | H |
| 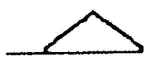 | H | 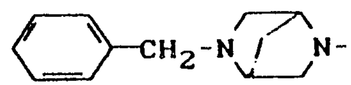 | $CH_3$ | H |

<u>Tabelle 1</u> (Fortsetzung)

| $R^1$ | $R^2$ | $R^4$ | $R^3$ | A |
|---|---|---|---|---|
| 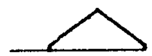 | H | 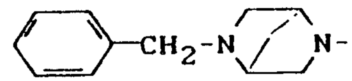 | $CH_3$ | F |
| 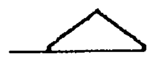 | H | 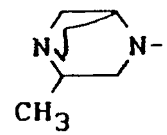 | $CH_3$ | $CH_3$ |
| 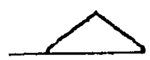 | H | 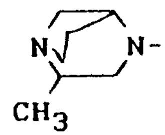 | $CH_3$ | CN |
| 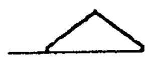 | H | 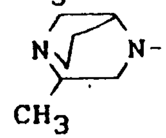 | $C_2H_5$ | F |
| 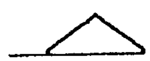 | H | 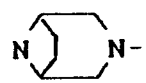 | $CH_3$ | Cl |
| 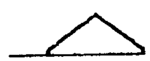 | H | 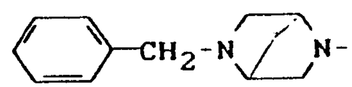 | $C_2H_5$ | Cl |

Tabelle 1 (Fortsetzung)

| $R^1$ | $R^2$ | $R^4$ | $R^3$ | A |
|---|---|---|---|---|
| $F-CH_2-CH_2$ | H | 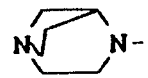 | $CH_3$ | F |
| 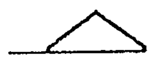 | H | 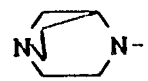 | $C_2H_5$ | $CH_3$ |
| 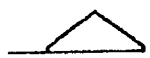 | H | 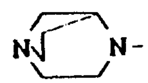 | $CH_3$ | $CH_3$ |
| 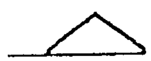 | H | 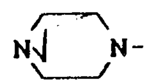 | $CH_3$ | $NO_2$ |
| $CH_3O-$ | H | 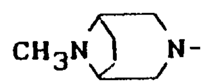 | $CH_3$ | H |

**Tabelle 1** (Fortsetzung)

| $R^1$ | $R^2$ | $R^4$ | $R^3$ | A |
|---|---|---|---|---|
| $CH_3-NH-$ | H | 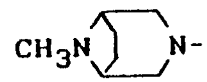 | $CH_3$ | F |
| 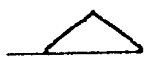 | H | 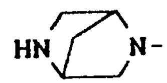 | $CH_3$ | Br |
| 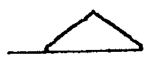 | H | 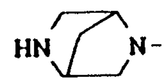 | $CH_3$ | CN |
| 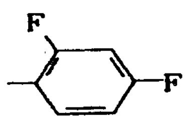 | H | 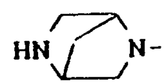 | $C_2H_5$ | CN |

**Tabelle 1** (Fortsetzung)

| $R^1$ | $R^2$ | $R^4$ | $R^3$ | A |
|---|---|---|---|---|
| 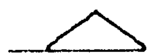 | H | 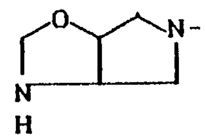 | $CH_3$ | $CH_3$ |
| 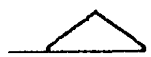 | $C_2H_5$ | 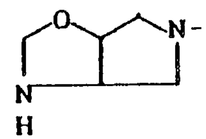 | $CH_3$ | H |
| 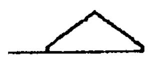 | H | 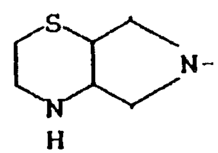 | $CH_3$ | H |
| 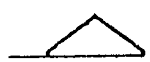 | H | 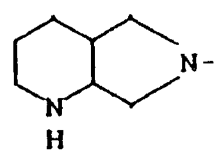 | $CH_3$ | H |

<u>Tabelle 1</u> (Fortsetzung)

| $R^1$ | $R^2$ | $R^4$ | $R^3$ | A |
|---|---|---|---|---|
| $C_2H_5$ | H | 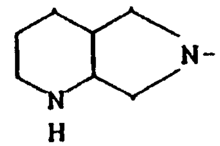 | $CH_3$ | H |
| $C_2H_5$ | H | 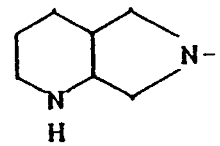 | $CH_3$ | F |
| 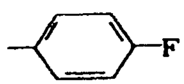 | H | 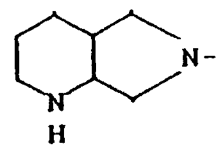 | $CH_3$ | H |
| 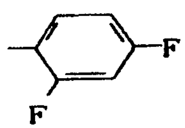 | H | 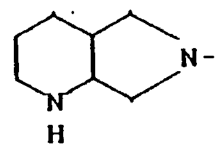 | $C_2H_5$ | F |
| $-CH=CH_2$ | H | 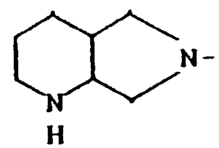 | $CH_3$ | H |

Tabelle 1 (Fortsetzung)

| $R^1$ | $R^2$ | $R^4$ | $R^3$ | A |
|---|---|---|---|---|
| $HO-CH_2CH_2-$ | H | 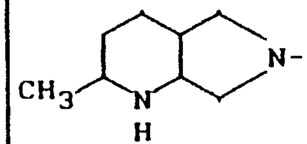 | $CH_3$ | H |
| 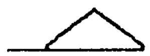 | H | 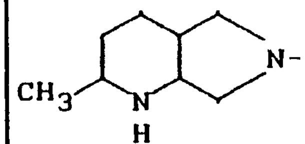 | $CH_3$ | F |
| 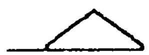 | H | 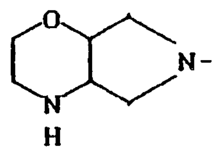 | $CH_3$ | Cl |
| 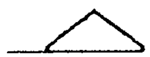 | $-C_2H_5$ | 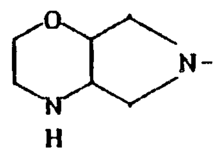 | $CH_3$ | Cl |
| 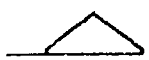 | 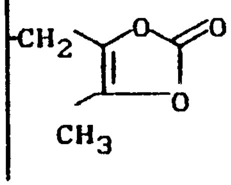 | 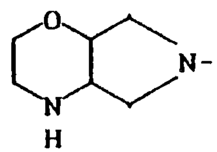 | $CH_3$ | F |

Tabelle 1 (Fortsetzung)

| $R^1$ | $R^2$ | $R^4$ | $R^3$ | A |
|---|---|---|---|---|
| 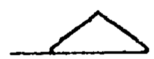 | H | 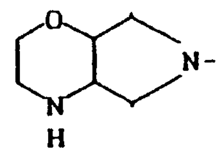 | $C_2H_5$ | H |
| 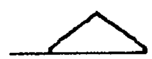 | H | 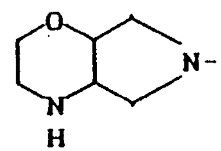 | $C_2H_5$ | F |
| 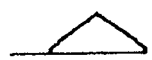 | H | 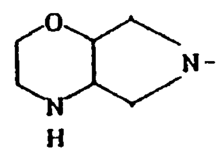 | $C_2H_5$ | Cl |
| 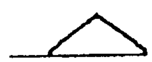 | H | 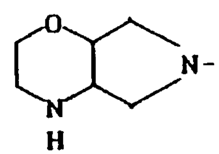 | $CH_3$ | CN |
| 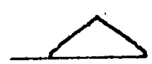 | H | 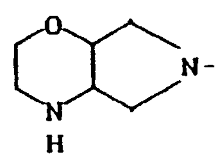 | $CH_3$ | $NO_2$ |

Tabelle 1 (Fortsetzung)

| $R^1$ | $R^2$ | $R^4$ | $R^3$ | A |
|---|---|---|---|---|
| 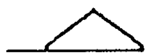 | H | 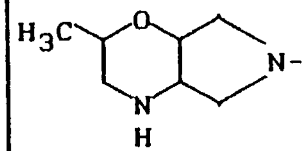 | $CH_3$ | $CH_3$ |
| 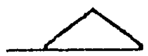 | H | 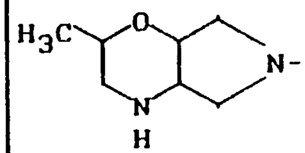 | $C_3H_7$ | H |
| 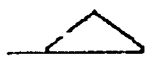 | H | 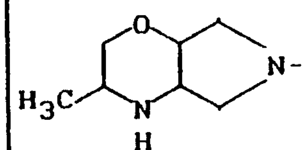 | $CH_3$ | F |
| 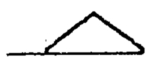 | H | 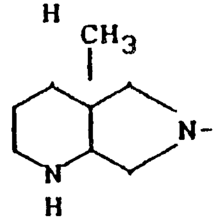 | $CH_3$ | Cl |
| $F-CH_2CH_2-$ | H | 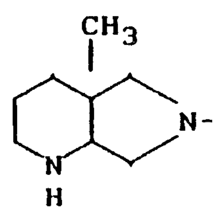 | $CH_3$ | F |

Tabelle 1 (Fortsetzung)

| $R^1$ | $R^2$ | $R^4$ | $R^3$ | A |
|---|---|---|---|---|
| 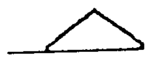 | H | 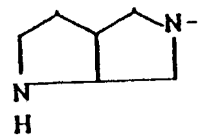 | $CH_3$ | H |
| 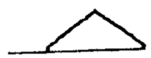 | H | 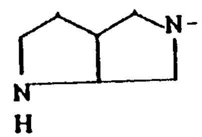 | $CH_3$ | F |
| 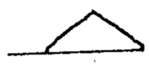 | H | 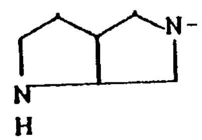 | $CH_3$ | Cl |
| $CH_3O$- | H | 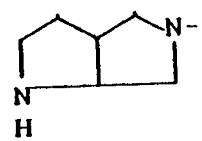 | $CH_3$ | H |
| $CH_3$-NH- | H | 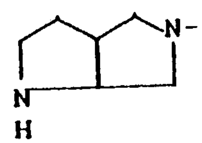 | $CH_3$ | H |

Tabelle 1 (Fortsetzung)

| $R^1$ | $R^2$ | $R^4$ | $R^3$ | A |
|---|---|---|---|---|
| 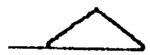 | H | 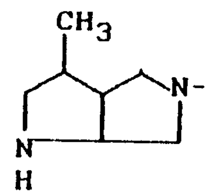 | $CH_3$ | H |
| 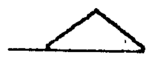 | H | 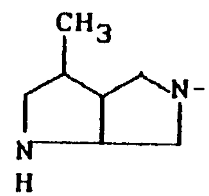 | $CH_3$ | F |
| 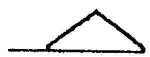 | H | 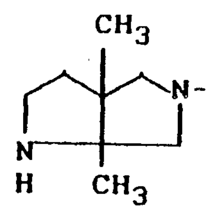 | $CH_3$ | Cl |
| 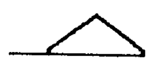 | H | 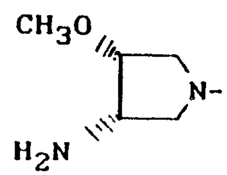 | $CH_3$ | F |

**Tabelle 1** (Fortsetzung)

| $R^1$ | $R^2$ | $R^4$ | $R^3$ | A |
|---|---|---|---|---|
| 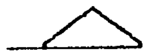 | H | 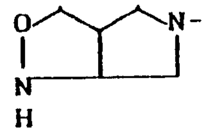 | $CH_3$ | H |
| 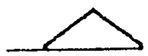 | H | 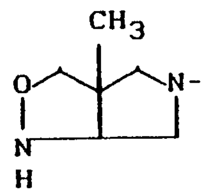 | $CH_3$ | F |
| 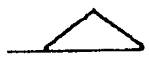 | H | 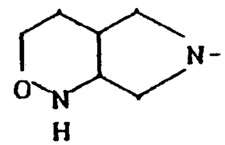 | $CH_3$ | H |
| 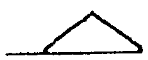 | H | 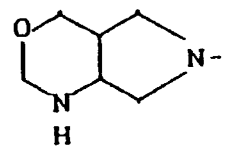 | $CH_3$ | F |
| 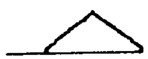 | H | 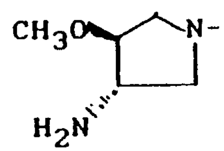 | $CH_3$ | $CH_3$ |

**Tabelle 1** (Fortsetzung)

| $R^1$ | $R^2$ | $R^4$ | $R^3$ | A |
|---|---|---|---|---|
| 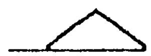 | H | 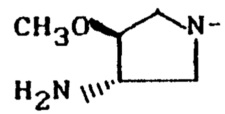 | $CH_3$ | F |
| 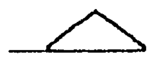 | H | 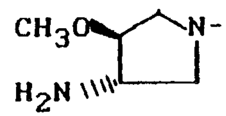 | $C_2H_5$ | F |
| $-C_2H_5$ | H | 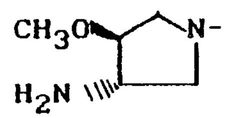 | $C_2H_5$ | Cl |
| 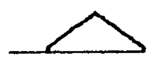 | $C_2H_5$ | 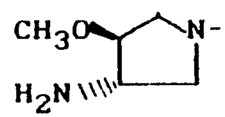 | $C_2H_5$ | Cl |
| 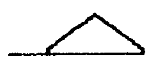 | H | 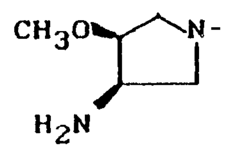 | $CH_3$ | F |

Tabelle 1 (Fortsetzung)

| $R^1$ | $R^2$ | $R^4$ | $R^3$ | A |
|---|---|---|---|---|
| 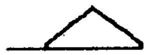 | $CH_3$ | 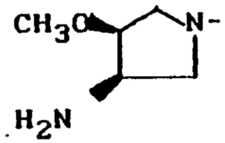 | $CH_3$ | Cl |
| $-C_2H_5$ | H | 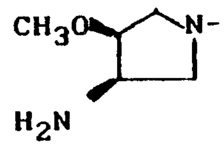 | $CH_3$ | F |
| 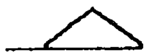 | H | 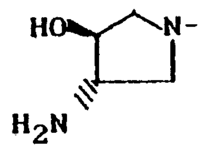 | $CH_3$ | F |
| 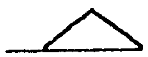 | H | 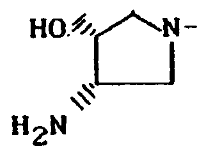 | $CH_3$ | F |
| 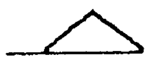 | H | 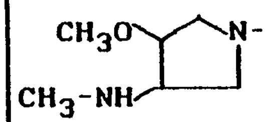 | $CH_3$ | H |

Tabelle 1 (Fortsetzung)

| $R^1$ | $R^2$ | $R^4$ | $R^3$ | A |
|---|---|---|---|---|
| 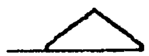 | H | 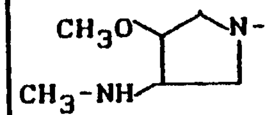 | $CH_3$ | F |
| 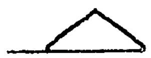 | H | 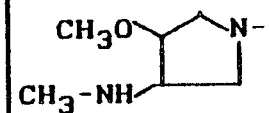 | $C_2H_5$ | F |
| 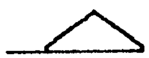 | H | 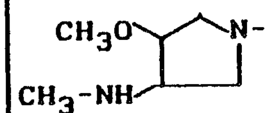 | $C_2H_5$ | Cl |
| 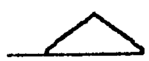 | H | 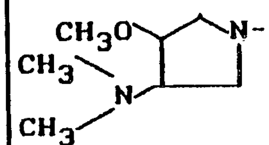 | $CH_3$ | F |
| 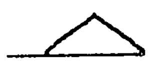 | H | 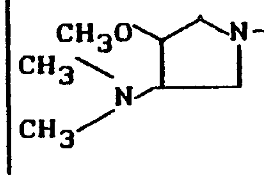 | $n-C_3H_7$ | H |

Tabelle 1 (Fortsetzung)

| $R^1$ | $R^2$ | $R^3$ | $R^4$ | A |
|---|---|---|---|---|
| 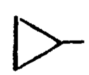 | H | $CH_3$ | 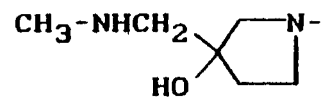 | H |
| " | H | $CH_3$ | " | F |
| " | H | $CH_3$ | " | Cl |
| " | $C_2H_5$ | $CH_3$ | " | F |
| " | 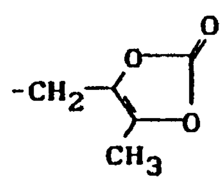 | $CH_3$ | " | F |
| " | H | $CH_3$ | " | CN |
| $C_2H_5$- | H | $CH_3$ | " | F |

Tabelle 1 (Fortsetzung)

| $R^1$ | $R^2$ | $R^3$ | $R^4$ | A |
|---|---|---|---|---|
| 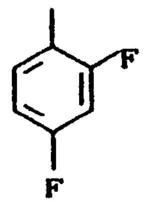 | H | $CH_3$ | 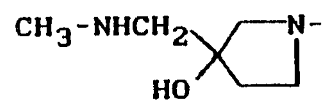 | F |
| 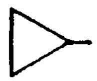 | H | $CH_3$ | 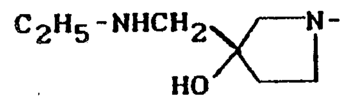 | H |
| " | H | $CH_3$ | " | F |
| " | H | $C_2H_5$ | " | F |
| $C_2H_5-$ | H | $CH_3$ | " | H |
| 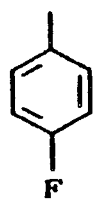 | H | $CH_3$ | " | H |
| 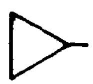 | $C_2H_5$ | $CH_3$ | " | F |

Tabelle 1 (Fortsetzung)

| $R^1$ | $R^2$ | $R^3$ | $R^4$ | A |
|---|---|---|---|---|
| 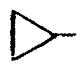 | 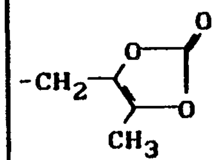 | $CH_3$ | 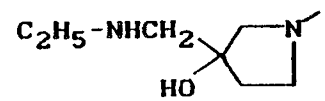 | H |
| " | $C_2H_5$ | $CH_3$ | " | F |
| " | 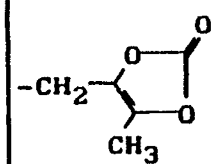 | $CH_3$ | " | F |
| " | H | $CH_3$ | 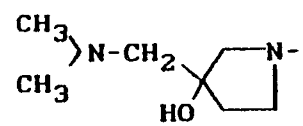 | H |
| " | H | $CH_3$ | " | F |
| " | H | $C_2H_5$ | " | F |
| " | H | $CH_3$ | " | Cl |
| " | H | $CH_3$ | 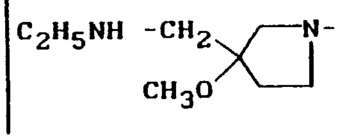 | H |

Tabelle 1 (Fortsetzung)

| $R^1$ | $R^2$ | $R^3$ | $R^4$ | A |
|---|---|---|---|---|
| 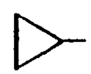 | H | $CH_3$ | 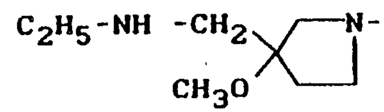 | F |
| " | H | $CH_3$ | " | Cl |
| " | H | $CH_3$ | 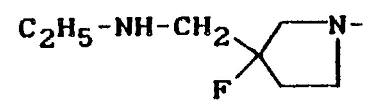 | F |
| " | H | $C_2H_5$ | " | F |
| " | H | $CH_3$ | " | Cl |
| " | H | $CH_3$ | " | CN |
| " | H | $CH_3$ | 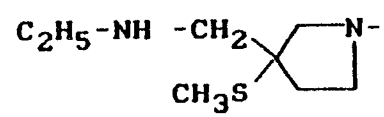 | H |
| " | H | $CH_3$ | " | F |

Tabelle 1 (Fortsetzung)

| $R^1$ | $R^2$ | $R^3$ | $R^4$ | A |
|---|---|---|---|---|
| 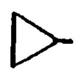 | H | $CH_3$ | 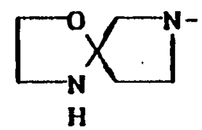 | H |
| " | H | $CH_3$ | " | F |
| " | H | $C_2H_5$ | " | F |
| " | H | $n-C_3H_7$ | " | F |
| " | H | $CH_3$ | " | Cl |
| " | H | $CH_3$ | 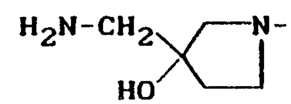 | F |
| " | H | $CH_3$ | " | $NO_2$ |
| " | H | $CH_3$ | " | CN |
| " | H | $CH_3$ | 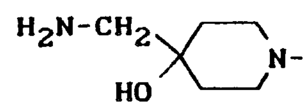 | F |
| " | H | $CH_3$ | " | H |
| " | H | $CH_3$ | " | Cl |

Tabelle 1 (Fortsetzung)

| $R^1$ | $R^2$ | $R^3$ | $R^4$ | A |
|---|---|---|---|---|
| 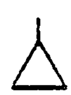 | H | $CH_3$ | 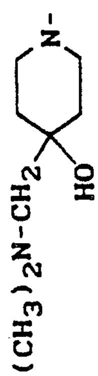 | H |
| " | H | $CH_3$ | " | F |

Beispiel A

7-Chlor-1-cyclopropyl-6,8-difluor-1,4-dihydro-5-methyl-4-oxo-3-chinolincarbonsäure

a)

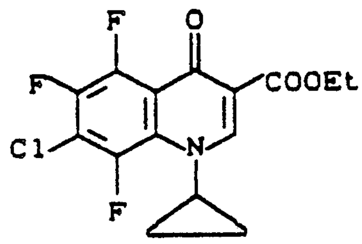

8,0 g 1-Cyclopropyl-5,6,7,8-tetrafluor-1,4-dihydro-4-oxo-3-chinolincarbonsäure und 7,9 ml Thionylchlorid werden gekocht, bis kein Gas mehr entweicht. Danach wird im Vakuum eingeengt. Dem Rückstand gibt man 50 ml Ethanol zu und kocht zwei Stunden.. Man kühlt auf Raumtemperatur ab und isoliert den ausgefallenen Feststoff.

Ausbeute: 8,6 g 7-Chlor-1-cyclopropyl-5,6,8-trifluor-1,4-dihydro-4-oxo-3-chinolincarbonsäureethylester
Schmelzpunkt: 166-168°

b)

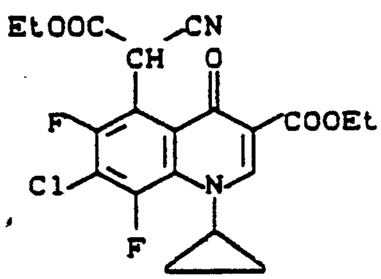

In 50 ml absolutem Dioxan legt man 1,6 ml (0,015 mol) Cyanessigsäureethylester vor und gibt bei 20°C 0,58 g Natriumhydrid (als 80%ige Ware) zu. Nach 30 Minuten gibt man 3,45 g (0,01 mol) Substanz aus a) zu. Danach wird 6 Stunden unter Rückfluß gekocht. Nach Abkühlen auf 20° wird mit Wasser verdünnt und mit Salzsäure sauer gestellt. Der Feststoff wird isoliert, getrocknet und aus Isopropanol umkristallisiert.

Ausbeute: 2,3 g 7-Chlor-5-(cyano-ethoxycarbonylmethyl)-1-cyclopropyl-6,8-difluor-1,4-dihydro-4-oxo-3-chinolincarbonsäureethylester
Schmelzpunkt: 156-57°.

c)

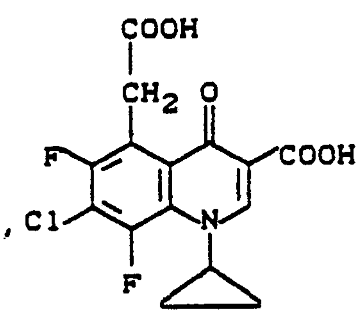

2,3 g Substanz aus b) werden zusammen mit 6 ml Essigsäure, 5 ml Wasser und 0,5 ml Schwefelsäure 4 Stunden auf 140° erhitzt. Man kühlt auf 20° ab und verdünnt mit Wasser. Der Feststoff wird isoliert, mit Wasser gewaschen und getrocknet.

Ausbeute: 1,6 g 5-Carboxymethyl-7-chlor-1-cyclopropyl-6,8-difluor-1,4-dihydro-4-oxo-3-chinolincarbonsäure

Ausbeute: 236-8° (Z)

| Analyse: | | | | |
|---|---|---|---|---|
| berechnet: | C 50,3 | H 3,0 | N 3,9 | Cl 9,9 |
| gefunden: | 50,5 | 3,2 | 3,7 | 10,0 |
| | 50,6 | 3,2 | 3,8 | 9,9 |

d)

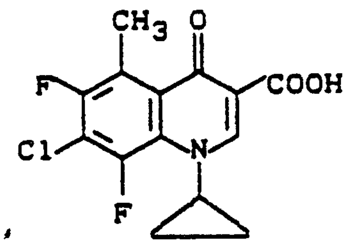

1,0 g (2,8 mmol) Substanz aus c) und 0,9 g (8,4 mmol) 1,4-Diazabicyclo[2,2,2]octan werden in 20 ml Dimethylsulfoxid 3 Stunden auf 140° erhitzt. Danach wird das Lösungsmittel im Hochvakuum entfernt und der Rückstand an Kieselgel (Laufmittel: Methylenchlorid/Methanol 99/1) chromatographiert.

Ausbeute: 0,2 g 7-Chlor-1-cyclopropyl-6,8-difluor-1,4-dihydro-5-methyl-4-oxo-3-chinolincarbonsäure
Schmelzpunkt: 195-7°

Beispiel 1

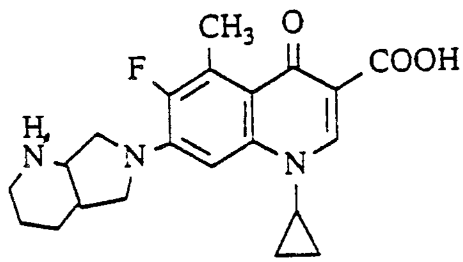

0,56 g (2 mmol) 1-Cyclopropyl-6,7-difluor-1,4-dihydro-5-methyl-4-oxo-3-chinolincarbonsäure werden mit 0,384 g (3 mmol) 2,8-Diazabicyclo[4,3,0]nonan und 0,672 g (6 mmol) 1,4-Diazabicyclo[2,2,2]octan in 3,5 ml Dimethylsulfoxid 2 Stunden auf 140° C erhitzt. Nach Abkühlen wird das DMSO im Hochvakuum entfernt. Der Rückstand wird mit Acetonitril aufgenommen. Man trennt den Feststoff ab, wäscht ihn mit Acetonitril und trocknet bei 60-80°.

Ausbeute: 0,7 g 1-Cyclopropyl-7-(2,8-diazabicyclo[4,3,0]non-8-yl)-6-fluor-1,4-dihydro-5-methyl-4-oxo-3-chinolincarbonsäure
Schmelzpunkt: 174-6° unter Zersetzung

Beispiel 2

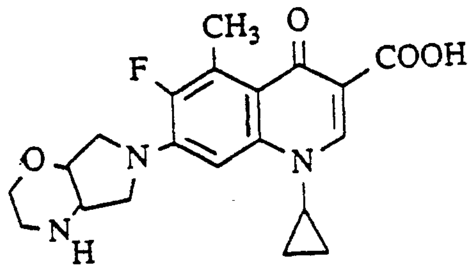

0,28 g (1 mmol) 1-Cyclopropyl-6,7-difluor-1,4-dihydro-5-methyl-4-oxo-3-chinolincarbonsäure werden in 3,5 ml Dimethylsulfoxid mit 0,19 g (1,5 mmol) 2-Oxa-5,8-diazabicyclo[4,3,0]nonan und 0,34 g (3 mmol) 1,4-Diazabicyclo-[2,2,2]octan 2 Stunden auf 140° C erhitzt. Das Dimethylsulfoxid wird im Hochvakuum abdestilliert. Der Rückstand wird mit Acetonitril verrührt und der Feststoff isoliert.

Ausbeute: 0,27 g 1-Cyclopropyl-6-fluor-1,4-dihydro-5-methyl-7-(2-oxa-5,8-diazabicyclo[4.3.0]non-8-yl)-4-oxo-3-chinolincarbonsäure
Schmelzpunkt: 273-5°

Beispiel 3

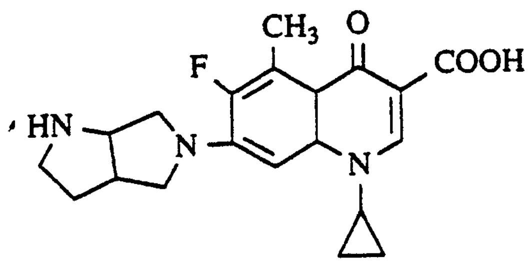

0,14 g (0,5 mmol) 1-Cyclopropyl-6,7-difluor-1,4-dihydro-5-methyl-4-oxo-3-chinolincarbonsäure werden in 3,5 ml Dimethylsulfoxid mit 0,084 g (0,75 mmol) 2,7-Diazabicyclo[3,3,0]octan und 0,17 g (1,5 mmol) 1,4-Diazabicyclo[2,2,2]octan 2 Stunden auf 140° erhitzt. Danach wird Dimethylsulfoxid im Hochvakuum abdestilliert. Dem Rückstand wird Acetonitril zugegeben, wobei sich ein Feststoff bildet.

Ausbeute: 0,15 g 1-Cyclopropyl-7-(2,7-diazabicyclo[3,3,0]oct-7-yl)-6-fluor-1,4-dihydro-5-methyl-4-oxo-3-chinolincarbonsäure
Schmelzpunkt: 232-4° unter Zersetzung

Beispiel 4

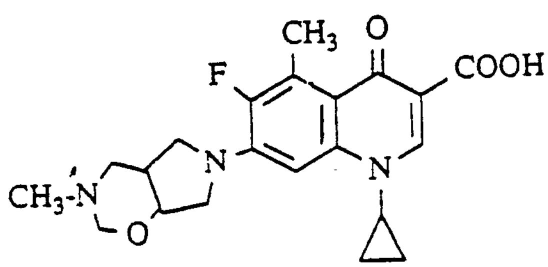

0,28 g (1 mmol) 1-Cyclopropyl-6,7-difluor-1,4-dihydro-5-methyl-4-oxo-3-chinolincarbonsäure werden in 3,5 ml Dimethylsulfoxid mit 0,213 g (1,5 mmol) 5-Methyl-3-oxo-5,8-diazabicyclo[4,3,0]nonan und 0,34 g (3 mmol) 1,4-Diazabicyclo[2,2,2]octan 2 Stunden auf 140° C erhitzt. Danach wird das Lösungsmittel im

Hochvakuum entfernt. Nach Verrühren des Rückstandes mit Acetonitril werden 0,22 g 1-Cyclopropyl-6-fluor-1,4-dihydro-5-methyl-7-(5-methyl-3-oxo-5,8-diazabicyclo[4,3,0]non-8-yl)-4-oxo-3-chinolincarbonsäure erhalten.

Schmelzpunkt: 208-10° unter Zersetzung

Beispiel 5

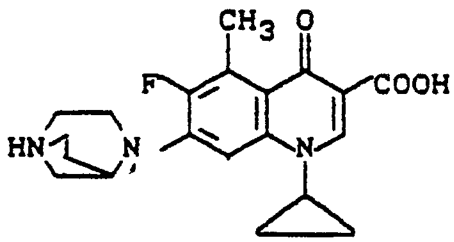

0,28 g (1 mmol) 1-Cyclopropyl-6,7-difluor-1,4-dihydro-5-methyl-4-oxo-3-chinolincarbonsäure werden in 3,5 ml Dimethylsulfoxid mit 0,17 g (1,5 mmol) 1,4-Diazabicyclo[3,2,1]octan und 0,34 g (3 mmol) 1,4-Diazabicyclo[2,2,2]octan 2 Stunden auf 140° erhitzt. Nach Entfernen des Lösungsmittels im Hochvakuum, wird der Rückstand mit Acetonitril verrührt und der Feststoff isoliert.

Ausbeute: 0,24 g 1-Cyclopropyl-7-(1,4-diazabicyclo[3,2,1]oct-4-yl)-6-fluor-1,4-dihydro-5-methyl-4-oxo-3-chinolincarbonsäure

Schmelzpunkt: 274-76° unter Zersetzung

Beispiel B

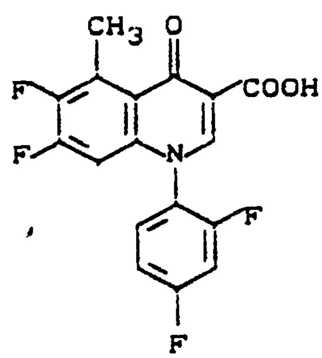

6,7-Difluor-1-(2,4-difluor-phenyl)-1,4-dihydro-5-methyl-4-oxo-3-chinolon-carbonsäure

a)

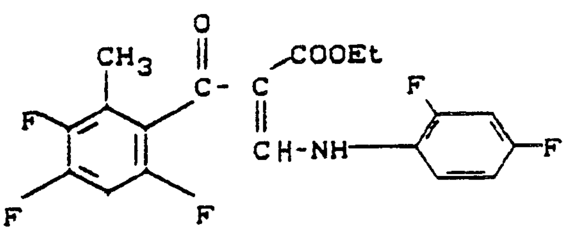

21 g 3-Ethoxy-2-(2,4,5-trifluor-6-methyl-benzoyl)-acrylsäureethylester werden in 55 ml Ethanol vorgelegt. Unter Kühlung werden 9,4 g 2,4-Difluoranilin zugetropft. Danach wird eine Stunde bei 25°C gerührt.

Man gibt anschließend 55 ml Wasser zu und isoliert den ausgefallenen Feststoff.

Ausbeute: 25 g 3-(2,4-Difluorphenylamino)-2-(2,4,5-trifluor-6-methyl-benzoyl)-acrylsäureethylester Schmelzpunkt 109-10 ° C.

b)

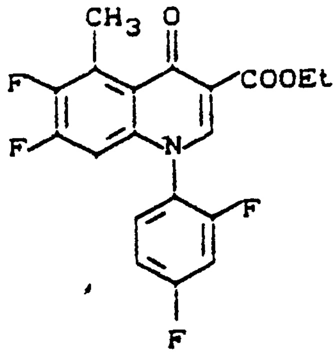

12,5 g Substanz aus a) und 5,1 g Kaliumcarbonat werden in 60 ml Dimethylformamid 4 Stunden auf 140 ° C erhitzt. Nach Abkühlung auf Raumtemperatur wird mit Wasser verdünnt. Der ausgefallene Feststoff wird isoliert und getrocknet.

Ausbeute: 11,3 g 6,7-Difluor-1-(2,4-difluorphenyl)-1,4-dihydro-5-methyl-4-oxo-3-chinolincärbonsäure-ethylester
Schmelzpunkt: 157-9 °

c)

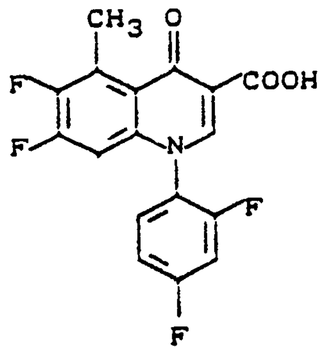

11,2 g Substanz aus b), 70 ml Essigsäure, 70 ml Wasser und 3,5 ml Schwefelsäure werden 4 Stunden auf 140 ° C erhitzt. Nach Abkühlen auf Raumtemperatur wird mit Wasser verdünnt. Der Feststoff wird isoliert und getrocknet. Man erhält 10,3 g der Titelverbindung. Schmelzpunkt: 277-8 ° C

Beispiel 6

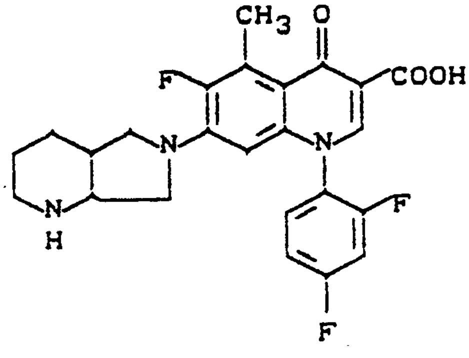

0,6 g Substanz aus Beispiel B, 0,57 g 1,4-Diazabicyclo[2,2,2]-octan und 0,25 g 2,8-Diazabicyclo[4,3,0]-nonan werden in 6 ml DMSO bei Raumtemperatur gerührt, bis im Dünnschichtchromatogramm kein Ausgangsmaterial mehr nachweisbar ist. Danach wird im Vakuum eingeengt. Den Rückstand versetzt man mit Wasser und isoliert den Feststoff.

Ausbeute: 0,6 g 7-(2,8-diazabicyclo[4,3,0]non-8-yl)-1-(2,4-difluorphenyl)-6-fluor-1,4-dihydro-5-methyl-4-oxo-3-chinolincarbonsäure.
Schmelzpunkt: 247-8° C.

**Ansprüche**

1. Chinoloncarbonsäurederivate der Formel (I)

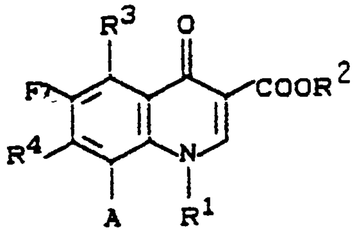

(I)

in welcher
$R^1$ für gegebenenfalls durch Hydroxy, Halogen, $C_1$-$C_3$-Alkoxy oder $C_1$-$C_3$-Alkylthio substiutiertes geradkettiges oder verzweigtes $C_1$-$C_4$-Alkyl, für gegebenenfalls durch Halogen oder $C_1$-$C_3$-Alkyl substituiertes $C_3$-$C_6$-Cycloalkyl, $C_1$-$C_4$-Alkenyl, ferner für $C_1$-$C_3$-Alkoxy, Amino, Monoalkylamino mit 1-3 C-Atomen, Dialkylamino und 2-6 C-Atomen oder für gegebenenfalls durch Halogen substituiertes Phenyl steht,
$R^2$ für Wasserstoff, Alkyl mit 1 bis 4 Kohlenstoffatomen oder (5-Methyl-2-oxo-1,3-dioxol-4-yl)-methyl steht
$R^3$ $C_1$-$C_4$-Alkyl bedeutet,
$R^4$ für einen im Ringsystem gegebenenfalls durch Hydroxy oder Methyl substituierten Rest der Formel

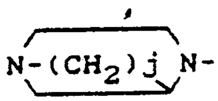 , 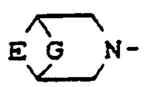 , 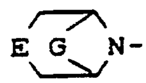 , 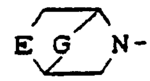 ,

in welcher
E für $R^5$-N, O, S,
G für $-(CH_2)_j-$, $-CH_2-O-CH_2-$,

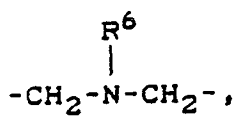

$-CH_2-S-CH_2-$, $-CH_2-S-$,
j für 1, 2 oder 3,
$R^5$ für Wasserstoff, gegebenenfalls durch Hydroxy substituiertes Alkyl, Alkenyl oder Alkinyl mit 1 bis 4 Kohlenstoffatomen, gegebenenfalls durch Nitro oder Amino substituiertes Benzyl, Oxoalkyl mit 2 bis 4 Kohlenstoffatomen oder (5-Methyl-)-2-oxo-1,3-dioxol-4-yl)-methyl,
$R^6$ für Wasserstoff oder Methyl steht,
$R^4$ ferner für einen Rest der Formel

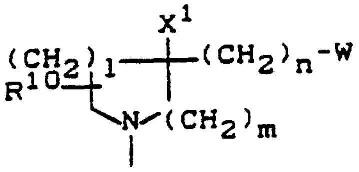

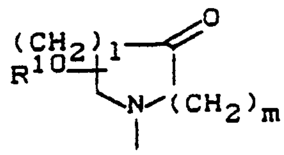

,

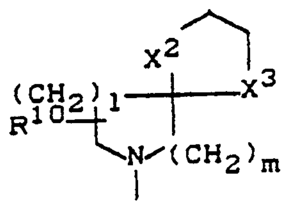

,

steht, worin
l für 0, 1 oder 2,
m für 1 oder 2 steht, wobei l + m gemeinsam 1, 2 oder 3 sein können,
n für 1 oder 2,
W für

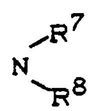

,

$OR^9$, $SR^9$, Halogen oder Wasserstoff,
$X^1$ für

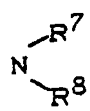

,

$OR^9$, $SR^9$, Halogen, CN, $CONH_2$, COOH oder $C_1$-$C_4$-Alkyl steht,
$X^2$ und $X^3$ gleich oder verschieden sein können und für Sauerstoff, Schwefel, NH oder N-$CH_3$ stehen,
$R^7$ für Wasserstoff, $C_1$-$C_3$-Alkyl, Allyl oder Propargyl und
$R^8$ für Wasserstoff, $C_1$-$C_3$-Alkyl oder $C_3$-$C_6$-Cycloalkyl steht,
wobei
$R^7$ + $R^8$ auch gemeinsam die Gruppen -$CH_2CH_2$-O-$CH_2CH_2$- oder -$(CH_2)_k$-, in welcher k für 3, 4, oder 5 stehen kann, bedeutet und
$R^9$ für Wasserstoff, $C_1$-$C_3$-Alkyl oder $C_1$-$C_3$-Acyl,
$R^{10}$ für Wasserstoff oder $C_1$-$C_3$-Alkyl steht,
$R^4$ ebenfalls einen Rest der Struktur

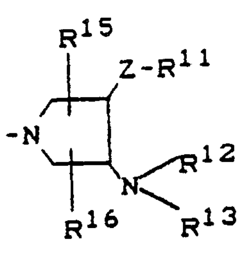

,

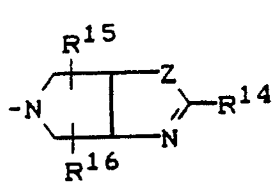

,

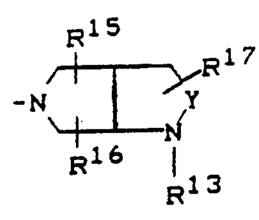

bedeutet, worin
$R^{11}$ für H, $C_1$-$C_3$-Alkyl, $C_1$-$C_2$-Acyl
$R^{12}$ für H, $C_1$-$C_3$-Alkyl, OH, $OCH_3$, wobei $R^{11}$ und $R^{12}$ gemeinsam auch eine gegebenenfalls durch Methyl ein- oder zweifach substituierte $C_1$-$C_3$-Alkylenbrücke bedeuten kann,
$R^{13}$ für H, $C_1$-$C_3$-Alkyl, Aryl, Heteroaryl, Benzyl, $C_1$-$C_4$-Alkoxycarbonyl, $C_1$-$C_4$-Acyl oder (5-Methyl-2-oxo-1,3-dioxol-4-yl)-methyl,

$R^{14}$ für H oder $C_1$-$C_4$-Alkyl,
$R^{15}$ für H, $CH_3$ oder Phenyl,
$R^{16}$ für H, $CH_3$ oder Phenyl,
$R^{17}$ für H oder $CH_3$,
Y für O, $CH_2$, $CH_2CH_2$ oder $CH_2$-O stehen kann, wobei die Verknüpfung der $CH_2$-O-Gruppe zum Stickstoff sowohl über O als auch über $CH_2$ erfolgen kann,
Z für O oder S stehen kann,
A für Wasserstoff, Halogen, Methyl, Cyano oder Nitro steht oder auch gemeinsam mit $R^1$ eine Brücke der Struktur
-O-$CH_2$ $\overset{*}{C}$H-$CH_3$ , -S-$CH_2$- $\overset{*}{C}$H-$CH_3$ oder -$CH_2CH_2$- $\overset{*}{C}$H-$CH_3$
mit R- bzw. S-Konfiguration bilden kann
und deren pharmazeutisch verwendbaren Hydrate und Säureadditionssalze sowie die Alkali-, Erdalkali-, Silber-und Guanidiniumsalze der zugrundeliegenden Carbonsäuren.

2. Verbindungen der Formel (I) gemäß Anspruch 1, in denen
$R^1$ für Ethyl, Isopropyl, Cyclopropyl, Vinyl, tert.-Butyl, 2-Hydroxyethyl, 2-Fluorethyl, Amino, Methylamino, Phenyl, 4-Fluorphenyl oder 2,4-Difluorphenyl,
$R^2$ für Wasserstoff, Alkyl mit 1 bis 3 Kohlenstoffatomen oder (5-Methyl-2-oxo-1,3-dioxol-4-yl)-methyl,
$R^3$ für $C_1$-$C_3$-Alkyl steht
$R^4$ für einen im Ringsystem gegebenenfalls durch Hydroxy oder Methyl substituierten Rest der Formel

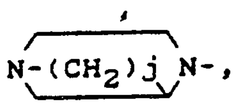, 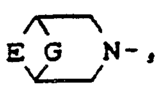, 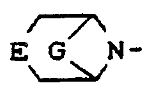, 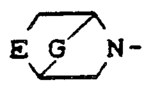 ,

in welcher
E für $R^5$-N, O, S,
G für $-(CH_2)_j-$, $-CH_2-O-CH_2-$,

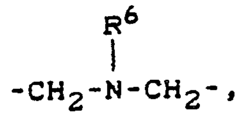

j für 1, 2 oder 3,
$R^5$ für Wasserstoff, gegebenenfalls durch Hydroxy substituiertes Alkyl, Alkenyl oder Alkinyl mit 1 bis 3 Kohlenstoffatomen, gegebenenfalls durch Nitro oder Amino substituiertes Benzyl oder Oxoalkyl mit 2 bis 4 Kohlenstoffatomen und
$R^6$ für Wasserstoff oder Methyl steht,
$R^4$ ferner für einen Rest der Formel

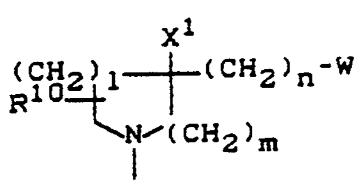

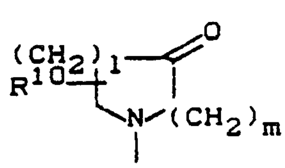

, 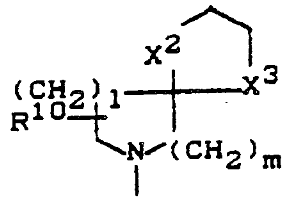 ,

steht worin,
l für 0, 1 oder 2,
m für 1 oder 2 steht, wobei l + m gemeinsam 1, 2 oder 3 sein können,

n für 1 oder 2,
W für

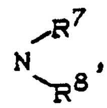

$OR^9$ oder Wasserstoff,
$X^1$ für

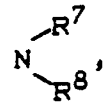

$OR^9$, Fluor, Chlor oder $C_1$-$C_2$-Alkyl steht,
$X^2$ und $X^3$ gleich oder verschieden sein können und für Sauerstoff, Schwefel oder $N$-$CH_3$ stehen,
$R^7$ für Wasserstoff, $C_1$-$C_2$-Alkyl oder Acetyl,
$R^8$ für Wasserstoff oder $C_1$-$C_2$-Alkyl steht,
wobei
$R^7$ + $R^8$ auch gemeinsam die Gruppen $-CH_2CH_2-O-CH_2CH_2-$ oder $-(CH_2)_k-$, in welcher k für 3, 4, oder 5 stehen kann, bedeutet,
steht,
$R^9$ für Wasserstoff, $C_1$-$C_2$-Alkyl oder Acetyl und
$R^{10}$ für Wasserstoff oder $C_1$-$C_2$-Alkyl steht,
$R^4$ ebenfalls einen Rest der Struktur

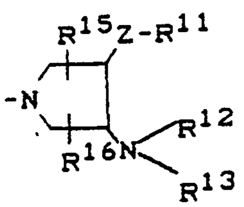 , 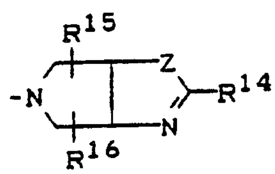 , 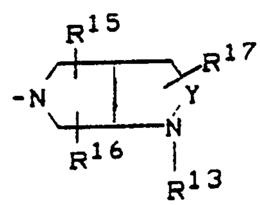

bedeutet, worin
$R^{11}$ für H, $C_1$-$C_3$-Alkyl, $C_1$-$C_2$-Acyl
$R^{12}$ für H, $C_1$-$C_3$-Alkyl, OH, $OCH_3$ steht, wobei $R^{11}$ und $R^{12}$ gemeinsam auch eine gegebenenfalls durch Methyl ein- oder zweifach substituierte $C_1$-$C_2$-Alkylenbrücke bedeuten kann,
$R^{13}$ für H, $C_1$-$C_3$-Alkyl, Hydroxyalkyl, Phenyl, Benzyl, $C_1$-$C_4$-Alkoxycarbonyl, $C_1$-$C_2$-Acyl oder (5-Methyl-2-oxo-1,3-dioxol-4-yl)-methyl,
$R^{14}$ für H oder $C_1$-$C_2$-Alkyl,
$R^{15}$ für H oder $CH_3$,
$R^{16}$ für H oder $CH_3$,
$R^{17}$ für H oder $CH_3$,
Y für O, $CH_2$, $CH_2CH_2$ oder $CH_2$-O stehen kann, wobei die Verknüpfung der $CH_2$-O-Gruppe zum Stickstoff sowohl über O als auch über $CH_2$ erfolgen kann,
Z für O,
A für H, Fluor, Chlor, Methyl, Cyano oder Nitro steht oder auch gemeinsam mit $R^1$ eine Brücke der Struktur
$-O-CH_2-\overset{*}{C}H-CH_3$
mit R- bzw. S-Konfiguration bilden kann.

3. Verbindungen dar Formel (I) gemäß Anspruch 1, in denen
$R^1$ für Ethyl, Vinyl, t-Butyl, Cyclopropyl, 2-Hydroxyethyl, 2-Fluorethyl, Methylamino, 4-Fluorphenyl, 2,4-Difluorphenyl,
$R^2$ für Wasserstoff, Alkyl mit 1 bis 2 Kohlenstoffatomen,
$R^3$ für $C_1$-$C_3$-Alkyl steht
$R^4$ für einen im Ringsystem gegebenenfalls durch Methyl substituierten Rest der Formel

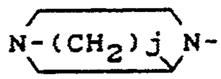, 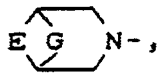, 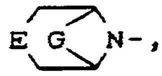, 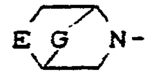 ,

in welcher
E für $R^5$-N,
G für $-(CH_2)_j-$,
j für 1 oder 2,
$R^5$ für Wasserstoff, gegebenenfalls durch Hydroxy substituiertes Alkyl, Alkenyl oder Alkinyl mit 1 bis 3 Kohlenstoffatomen, gegebenenfalls durch Nitro oder Amino substituiertes Benzyl oder Oxoalkyl mit 2 bis 4 Kohlenstoffatomen und
$R^6$ für Wasserstoff oder Methyl steht,
$R^4$ ferner für einen Rest der Formel

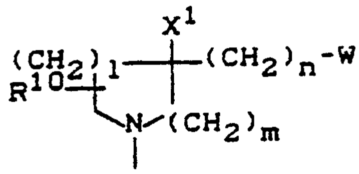

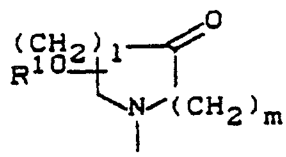

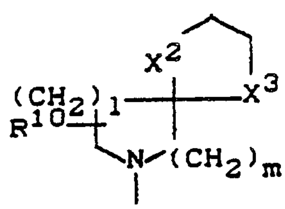 ,

worin
l für 0, 1 oder 2,
m für 1 oder 2 steht, wobei l + m gemeinsam 1, 2 oder 3 sein können,
n für 1,
W für

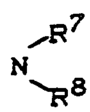,

$OR^9$, oder Wasserstoff,
$X^1$ für

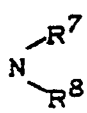 ,

$OR^9$, Chlor oder Alkyl steht
$X^2$ und $X^3$ gleich oder verschieden sein können und für Sauerstoff oder $N-CH_3$ stehen,
$R^7$ für Wasserstoff oder Methyl und
$R^8$ für Wasserstoff oder Methyl steht,
wobei
$R^7$ + $R^8$ auch gemeinsam die Gruppen $-CH_2CH_2-O-CH_2CH_2-$ oder $-(CH_2)_k-$, in welcher k für 3, 4, oder 5 stehen kann, bedeutet und
$R^9$ für Wasserstoff, $C_1$-$C_3$-Alkyl oder $C_1$-$C_3$-Acyl,
$R^{10}$ für Wasserstoff oder $C_1$-$C_3$-Alkyl steht,
$R^4$ ebenfalls einen Rest der Struktur

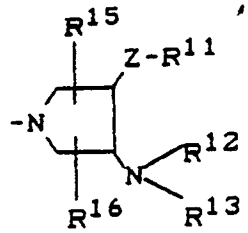, 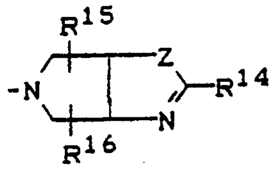, 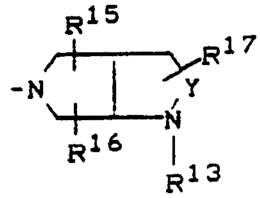

bedeutet, worin
$R^{11}$ für H, $C_1$-$C_2$-Alkyl, Acetyl,
$R^{12}$ für H, $C_1$-$C_2$-Alkyl, wobei $R^{11}$ und $R^{12}$ gemeinsam auch eine gegebenenfalls durch Methyl substituierte $C_1$-$C_2$-Alkylenbrücke bedeuten kann,
$R^{13}$ für H, $C_1$-$C_2$-Alkyl, Hydroxyethyl, Benzyl, $C_1$-$C_4$-Alkoxycarbonyl, $C_1$-$C_2$-Acyl,
$R^{14}$ für H oder $CH_3$,
$R^{15}$ für H oder $CH_3$,
$R^{16}$ für H oder $CH_3$,
$R^{17}$ für H oder $CH_3$,
Y für O, $CH_2$, $CH_2CH_2$ oder $CH_2$-O stehen kann, wobei die Verknüpfung der $CH_2$-O-Gruppen zum Stickstoff sowohl über O als auch über $CH_2$ erfolgen kann,
Z für O stehen kann,
A für H, Fluor oder Chlor steht, oder auch gemeinsam mit $R^1$ eine Brücke der Struktur $-O-CH_2-\overset{*}{C}H-CH_3$ mit R- bzw. S-Konfiguration bilden kann.

4. Verfahren zur Herstellung von Verbindungen der Formel (I) gemäß Anspruch 1, dadurch gekennzeichnet, daß man Verbindungen der Formel (II)

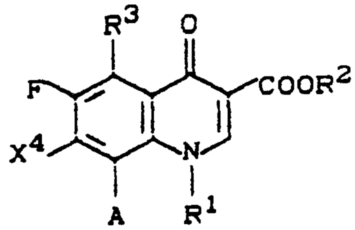 (II)

in welcher
$R^1$, $R^2$, $R^3$ und A die in Anspruch 1 angegebene Bedeutung haben und
$X^4$ für Halogen, insbesondere Fluor oder Chlor steht, mit Verbindungen der Formel (III)
$R^4$-H (III)
in welcher
$R^4$ die in Anspruch 1 angegebene Bedeutung hat,
gegebenenfalls in Gegenwart von Säurefängern umsetzt und gegebenenfalls in $R^4$ enthaltene Schutzgruppen abspaltet.

5. Verbindungen der Formel (I) gemäß Anspruch 1 zur Anwendung in einem Verfahren zur therapeutischen Behandlung des menschlichen oder tierischen Körpers.

6. Arzneimittel, enthaltend Verbindungen der Formel (I) gemäß Anspruch 1.

7. Verwendung von Verbindungen der Formel (I) gemäß Anspruch 1 zur und bei der Herstellung von Arzneimitteln.

8. 7-Chlor-1-cyclopropyl-6,8-difluor-1,4-dihydro-5-methyl-4-oxo-3-chinolincarbonsäure.

Europäisches Patentamt

# EUROPÄISCHER RECHERCHENBERICHT

Nummer der Anmeldung

EP 90105293.6

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int Cl⁵) |
| P,X | EP - A2 - 0 326 891 (WARNER-LAMBERT COMPANY) * Ansprüche 1,11-13 * -- | 1-8 | C 07 D 487/08<br>C 07 D 471/04<br>C 07 D 498/04<br>C 07 D 487/04<br>C 07 D 401/04<br>A 61 K 31/47 //<br>(C 07 D 487/08,<br>C 07 D 209:00<br>C 07 D 209:00)<br>(C 07 D 487/08,<br>C 07 D 243:00<br>C 07 D 209:00)<br>(C 07 D 471/04,<br>C 07 D 221:00<br>C 07 D 209:00)<br>(C 07 D 498/04,<br>C 07 D 263:00,<br>C 07 D 209:00)<br>(C 07 D 498/04, |
| X | EP - A2 - 0 287 951 (OTSUKA PHARMACEUTICAL CO.) * Ansprüche 1,44-46; Seiten 5-9 * -- | 1-8 | |
| X | EP - A1 - 0 276 700 (BAYER AG) * Ansprüche 1,5-7 * -- | 1-8 | |
| X | EP - A2 - 0 206 076 (BAYER AG) * Ansprüche 1,3,7,8 * -- | 1-8 | |
| X | EP - A2 - 0 225 552 (BAYER AG) * Ansprüche 1,6,9,10 * -- | 1-8 | RECHERCHIERTE SACHGEBIETE (Int Cl⁵) |
| D,A | EP - A2 - 0 230 274 (BAYER AG) * Ansprüche 1,5,10,11 * ---- | 1-8 | C 07 D 487/00<br>C 07 D 471/00<br>C 07 D 498/00<br>C 07 D 513/00<br>C 07 D 455/00<br>C 07 D 519/00<br>C 07 D 215/00<br>C 07 D 401/00 |

Der vorliegende Recherchenbericht wurde für alle Patentansprüche erstellt.

| Recherchenort | Abschlußdatum der Recherche | Prüfer |
|---|---|---|
| WIEN | 28-05-1990 | PETROUSEK |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches Patentamt

# EUROPÄISCHER RECHERCHENBERICHT

Nummer der Anmeldung

-2-

EP 90105293.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| | | | C 07 D 265:00, C 07 D 209:00) (C 07 D 487/04, C 07 D 209:00, C 07 D 209:00) |
| | | | RECHERCHIERTE SACHGEBIETE (Int Cl 5) |
| | | | |

Der vorliegende Recherchenbericht wurde für alle Patentansprüche erstellt.

| Recherchenort | Abschlußdatum der Recherche | Prüfer |
|---|---|---|
| WIEN | 28-05-1990 | PETROUSEK |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument